# EUROPEAN PATENT APPLICATION

(11) **EP 4 641 196 A1**
(43) Date of publication of application: **29.10.2025**
(21) Application number: 23905840.7
(22) Date of filing: 15.12.2023
(51) Int. Cl.: G01N 33/53

(54) **SYSTEM AND METHOD FOR SCREENING RNA APTAMER**

(30) Priority: 19.12.2022 CN 202211635692
(71) Applicant: CRISMERS BIOTECHNOLOGIES (SHENZHEN) CO., LTD., Shenzhen, Guangdong 518081 (CN)
(72) Inventor: WANG, Yu, Shenzhen, Guangdong 518055 (CN); ZHANG, Ju, Shenzhen, Guangdong 518055 (CN)
(74) Representative: Kuttenkeuler, David
(86) International application number: PCT/CN2023/139189
(87) International publication number: WO 2024/131676

(57) **Abstract**

Disclosed is a CRISPR/Cas based system for screening an RNA aptamer against a target protein, comprising: (i) a guide RNA comprising a recognition sequence and a nucleic acid aptamer random library having a predetermined length; (ii) a target sequence complementary to the recognition sequence of the guide RNA; (iii) a selection marker located downstream of the target sequence and comprising a basal promoter and a selection marker gene; (iv) a fusion protein comprising the target protein and a transcriptional activation module, wherein a binding of the target protein to an RNA aptamer of the nucleic acid aptamer random library results in recruitment of the transcriptional activation module to the selection marker; (v) a dCas protein specifically recognizing the target sequence under the guidance of the guide RNA; and (vi) a screening cell. Also disclosed is a method for screening an RNA aptamer against a target protein using the system provided herein and RNA aptamers against S1 protein of SARS-CoV-2 virus and applications thereof.

## Description

### Technical Filed

The present disclosure relates to a system and method for screening RNA aptamers, and in particular to such a system and method based on CRISPR/Cas technology. The present disclosure also relates to RNA aptamers, in particular, RNA aptamers for detecting or neutralizing SARS-CoV-2 virus and applications thereof.

### Background

Systematic evolution of ligands by exponential enrichment (SELEX) has long been the gold standard for screening ssDNA or RNA aptamers since 1990. In general, one cycle of SELEX includes the following steps: construction of a nucleotide sequence library, acquisition of the target molecules, incubation, affinity selection, and recovery of binding complexes, amplification and analysis of recovered nucleotide sequences. Ultimately, specific affinity sequences, namely the aptamers, will dominate the remaining library population and be identified. Subsequent exploratory work, including functional validation and chemical modifications, will then be conducted. The final selected aptamers are highly sensitive to the screening environment and experimental parameters, such as the ratio of screening library to target molecule, buffer composition, ionic strength, pH, incubation temperature, incubation duration, and intrinsic properties of the target molecule. The cumulative effect of these screening variables imposes a specific selection stringency, thereby critically influencing the true affinity and efficacy of the obtained aptamers.

Purified-protein-based SELEX may not simulate the native conformations of target proteins. Cell-based SELEX screening can only efficiently target proteins on the cell surface, which limits the proportion of applicable proteins. Animal-based SELEX screening process is costly with a lack of specificity and throughput and could become complicated due to *in vivo* environment.

Currently, promising nucleic acid aptamers remain limited and fall far short of meeting practical requirements. Efficient RNA aptamer screening methods are highly desirable in the field.

Nucleic acid aptamers have found versatile applications across diverse biomedical domains, primarily encompassing therapeutic interventions, biosensor technologies, molecular imaging and in vitro diagnostics, biomarker discovery technologies, drug delivery systems, as well as antiviral and vaccine-based gene therapy strategies. The inherent capacity of aptamers for conjugation, labeling, and chemical modification facilitates their seamless integration with emerging technologies including endogenous nucleic acid analysis, microfluidic cell sorting, flow cytometry, and nanoparticle engineering, thereby enhancing diagnostic efficacy and advancing clinical research endeavors.

In the wake of the global outbreak of the COVID-19 pandemic, research institutions and pharmaceutical enterprises worldwide have intensified collaborative efforts in SARS-CoV-2-targeted drug screening and vaccine development. This global health crisis has underscored the urgent need to develop additional preventive and/or therapeutic pharmacological agents and methodologies for COVID-19.

### Summary

One aspect of the present invention provides a system for screening an RNA aptamer against a target protein, comprising: (i) a guide RNA comprising a recognition sequence and a nucleic acid aptamer random library having a predetermined length; (ii) a target sequence complementary to the recognition sequence of the guide RNA; (iii) a selection marker located downstream of the target sequence and comprising a basal promoter and a selection marker gene; (iv) a fusion protein comprising the target protein and a transcriptional activation module, wherein a binding of the target protein to an RNA aptamer of the nucleic acid aptamer random library results in recruitment of the transcriptional activation module to the selection marker; (v) a dCas protein specifically recognizing the target sequence under the guidance of the guide RNA; and (vi) a screening cell. In some embodiments, the nucleic acid aptamer random library consists of single-stranded oligonucleotide random sequences having 8 to 60 bases in the order of 10⁵ to 10³⁶. In some embodiments, the nucleic acid aptamer random library is inserted into a loop on the guide RNA scaffold. In some embodiments, the target sequence is a heterologous sequence and/or an artificial sequence. In some embodiments, the target sequence comprises a plurality of copies of a gLuc sequence or Tet sequence. In some embodiments, the selection marker gene is a luciferase gene, a fluorescent protein gene or an antibiotic resistance gene. In some embodiments, the luciferase gene is a firefly luciferase gene, renilla luciferase or a gaussia luciferase gene. In some embodiments, the antibiotic resistance gene is a puromycin resistance gene or a kanamycin resistance gene. In some embodiments, the fluorescent protein gene is a green fluorescent protein gene or a red fluorescent protein gene. In some embodiments, the transcriptional activation module comprises a plurality of transcription activation factors. In some embodiments, the transcriptional activation module comprises VP64, P65 and HSF1. In some embodiments, the basal promoter is selected from mini-promoter-1 (SEQ ID NO: 28), mini-promoter-2 (SEQ ID NO: 27), mini-TK promoter (SEQ ID NO: 29), mini-CMV promoter (SEQ ID NO: 30) and Crystallin basal promoter (SEQ ID NO: 31). In some embodiments, the dCas protein is a dCas9 protein or dUn1Cas12f1 protein. In some embodiments, the target protein is derived from a virus, a bacterial, a fungus or an animal. In some embodiments, the target protein is derived from a human. In some embodiments, the target protein is a green fluorescent protein or is derived from a RBD region of S1 protein from SARS-CoV-2. In some embodiments, the screening cell is a HEK293T cell or *E. coli.*

Another aspect of the invention provides a system for screening an RNA aptamer against a target protein, comprising a viral expression vector, wherein the viral expression vector comprises a guide RNA and a nucleic acid encoding a fusion protein, wherein the guide RNA comprises a recognition sequence and a nucleic acid aptamer random library having a predetermined length; the fusion protein comprises the target protein and a transcriptional activation module, wherein a binding of the target protein to an RNA aptamer of the nucleic acid aptamer random library results in recruitment of the transcriptional activation module to the selection marker; and a screening cell that expresses the dCas protein, wherein the screening cell comprises a target sequence and a selection marker, wherein the target sequence is complementary to the recognition sequence of the guide RNA; the selection marker is downstream of the target sequence and comprises a basal promoter and a selection marker gene. In some embodiments, the viral expression vector is a lentiviral expression vector. In some embodiments, the lentiviral expression vector further comprises a first promoter operably linked to the guide RNA and a second promoter operably linked to the nucleic acid encoding the fusion protein. In some embodiments, the nucleic acid aptamer random library is inserted into the scaffold of the guide RNA through a first restriction enzyme. In some embodiments, a nucleic acid encoding the target protein is operably linked to the second promoter through a second restriction enzyme. In some embodiments, the lentiviral expression vector comprises a polyT downstream of the guide RNA. In some embodiments, the lentiviral expression vector comprises a nuclear localization sequence (NLS). In some embodiments, the NLS comprises a first NLS downstream of the second promoter and a second NLS downstream of the nucleic acid encoding the fusion protein. In some embodiments, the lentiviral expression vector comprises P2A, an antibiotic resistance gene and a post-transcription regulation element downstream of the second NLS. In some embodiments, the P2A is linked to the second NLS in the upstream and the antibiotic resistance gene in the downstream, respectively, through a linker; and the nucleic acid encoding the target protein is linked to the first NLS in the upstream and a nucleic acid encoding the transcription activation factor in the downstream, respectively, through a linker.

Another aspect of the present invention provides a system for screening an RNA aptamer against a target protein, comprising one or more plasmid vectors comprising one or more selected from a group consisting of: a guide RNA; a target sequence and a selection marker; a nucleic acid sequence encoding a fusion protein; and a nucleic acid sequence encoding a dCas protein, wherein the guide RNA comprises a recognition sequence and a nucleic acid aptamer random library having a predetermined length, the target sequence is complementary to the recognition sequence of the guide RNA, the selection marker is downstream of the target sequence and comprises a basal promoter and a selection marker gene, the fusion protein comprises the target protein and a transcriptional activation module, wherein a binding of the target protein to an RNA aptamer of the nucleic acid aptamer random library results in recruitment of the transcriptional activation module to the selection marker, and the dCas protein specifically recognizes the target sequence under the guidance of the guide RNA; and a screening cell. In some embodiments, the screening cell is a prokaryotic cell.

Another aspect of the present invention provides a method for screening an RNA aptamer against a target protein, comprising: (1) providing a screening cell, wherein the screening cell comprises a guide RNA, a target sequence, a selection marker, a fusion protein and a dCas protein, wherein the guide RNA comprises a recognition sequence and a nucleic acid aptamer random library having a predetermined length; the target sequence is complementary to the recognition sequence of the guide RNA; the selection marker is downstream of the target sequence and comprises a basal promoter and a selection marker gene; the fusion protein comprises the target protein and a transcriptional activation module, wherein a binding of the target protein to an RNA aptamer of the nucleic acid aptamer random library results in recruitment of the transcriptional activation module to the selection marker; and the dCas protein specifically recognizes the target sequence under the guidance of the guide RNA; (2) screening the screening cell using the selection marker gene; (3) collecting the screening cell that expresses the selection marker gene; and (4) lysing the screening cell, specifically amplifying a region containing the nucleic acid aptamer, and obtaining sequence information regarding the nucleic acid aptamer by sequencing. In some embodiments, step (4) further comprises an analysis and verification of the nucleic acid aptamer as obtained. In some embodiments, the screening cell is obtained by transforming one or more plasmid vectors into a prokaryotic cell, wherein the one or more plasmid vectors comprise one or more selected from a group consisting of: the guide RNA; the target sequence and the selection marker; a nucleic acid sequence encoding the fusion protein; and a nucleic acid sequence encoding the dCas protein. In some embodiments, the prokaryotic cell is *E. coli.* In some embodiments, the screening cell is obtained through the following steps: (1.1) providing a cell that expresses the dCas protein, wherein the cell further comprises the target sequence and the selection marker; (1.2) providing a viral expression vector comprising a nucleic acid encoding the guide RNA and the fusion protein; and (1.3) packaging the viral expression vector and infecting the cell with the packaged viral expression vector. In some embodiments, the viral expression vector is lentiviral expression vector.

Another aspect of the present invention provides an RNA aptamer specifically binding to S1 protein of SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7.

Another aspect of the present invention provides a detection reagent or kit for detecting a SARS-CoV-2 virus, wherein the detection reagent or kit comprises an RNA aptamer specifically binding to S1 protein of the SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7.

Another aspect of the present invention provides a method for detecting a SARS-CoV-2 virus, comprising contacting a sample to be detected with an RNA aptamer specifically binding to S1 protein of the SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7.

Another aspect of the present invention provides use of an RNA aptamer specifically binding to S1 protein of a SARS-CoV-2 virus in the preparation of a detection reagent or kit for detecting a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7.

Another aspect of the present invention provides a pharmaceutical agent comprising an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a method for neutralizing a SARS-CoV-2 virus in a subject, comprising administering to the subject in need thereof an effective amount of the pharmaceutical agent provided herein, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides use of the pharmaceutical agent as provided herein in the preparation of a medicine for neutralizing a SARS-CoV-2 virus in a subject, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a method for prevention or treatment of a SARS-CoV-2 virus infection, comprising administering to a subject in need thereof an effective amount of the pharmaceutical agent as provided herein, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides use of the pharmaceutical agent as provided herein in the preparation of a medicine for prevention or treatment of a SARS-CoV-2 virus infection in a subject, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a pharmaceutical composition comprising the pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a method for neutralizing a SARS-CoV-2 virus in a subject, comprising administering to a subject in need thereof an effective amount of the pharmaceutical composition as provided herein, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides use of a pharmaceutical composition as provided herein in the preparation of a medicine for neutralizing a SARS-CoV-2 virus in a subject, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a method for prevention or treatment of SARS-CoV-2 virus infection in a subject, comprising administering to the subject in need thereof an effective amount of a pharmaceutical composition as provided herein, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides use of a pharmaceutical composition as provided herein in the preparation of a medicine for prevention or treatment of SARS-CoV-2 virus infection in a subject, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the pharmaceutical agent further comprises a viral or non-viral vector, wherein the vector comprises or carries the RNA aptamer specifically binding to S1 protein from the SARS-CoV-2 virus.

Another aspect of the present invention provides a pharmaceutical kit for neutralizing a SARS-CoV-2 virus in a subject, wherein the pharmaceutical kit comprises, independently, two or more pharmaceutical agents or pharmaceutical compositions as provided herein.

Another aspect of the present invention provides a pharmaceutical kit for prevention or treatment of SARS-CoV-2 infection in a subject, wherein the pharmaceutical kit comprises, independently, two or more pharmaceutical agents or pharmaceutical compositions as provided herein.

### Brief Description of the Figures

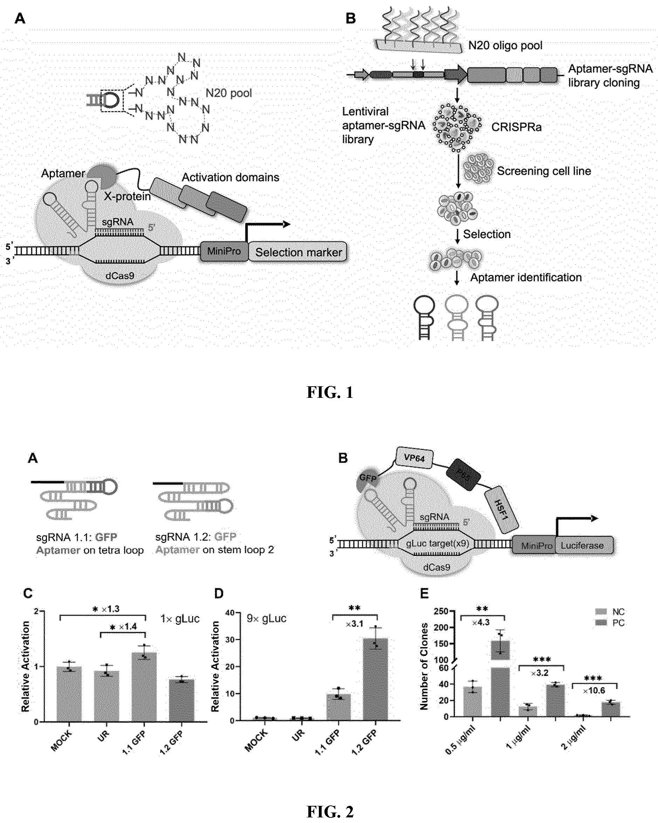

**Figure 1****.** CRISPR/Cas9-based nucleic acid aptamer screening system. (A) Schematic illustration of a molecular device repurposing CRISPR/Cas9 system to screen RNA aptamers. (B) Screening process of the RNA aptamer screening system. The aptamer is colored in red; the protein of interest (X-protein) is colored in orange; the transcription activation domains are colored in green; the mini promoter is colored in blue; and the selection marker is colored in pink.
**Figure 2****.** Validation of the nucleic acid aptamer screening system. (A) a GFP aptamer was incorporated in the tetraloop of sgRNA scaffold (sgRNA 1.1) and the stem loop2 of sgRNA scaffold (sgRNA 1.2), respectively. (B) Illustration of the luciferase reporter assay to detect the affinity binding of an aptamer to its GFP target. (C) Results of a luciferase assay shown in (B), using a single copy of gLuc sgRNA target sequence (1 x gLuc) to test the sensitivity. (D) Results of a luciferase assay shown in (B), using 9 repeats of gLuc sgRNA target sequences (9 x gLuc) to amplify the sensitivity. (E) Quantification of the number of clones survived upon lentiviral delivery of sgRNA 1.2-GFP aptamer or sgRNA 1.2-random aptamer and GFP-VPH into No. 4-53 monoclonal dCas9/9 x gLuc-Puro cell line. The Positive Control/Negative Control (PC/NC) ratios were shown on the top and the puromycin concentration used was indicated at the bottom. Mock: transient transfection of control plasmids. UR: control with random sequences. NC: negative control with sgRNA 1.2-random aptamer appendage and GFP-VPH. PC: positive control with sgRNA 1.2-GFP aptamer and GFP-VPH. Data was shown in mean ± SD in three biological replicates. **P* < 0.05, ***P<* 0.01, ****P* < 0.001, two-tailed t-tests.

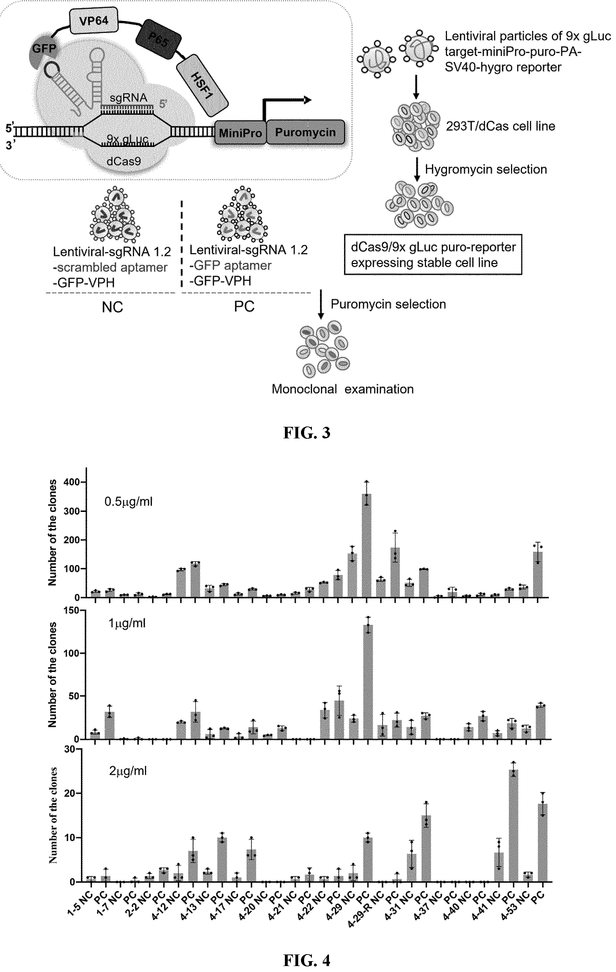

**Figure 3****.** Schematic illustration of the gLuc puromycin reporter assay for testing the binding affinity of GFP aptamers to GFP target protein and the screening and identifying process of dCas9/9 x gLuc-puro monoclonal cell lines.
**Figure 4****.** Results from a screen of dCas9/9 x gLuc-puro monoclonal cell lines by quantifying the numbers of surviving clones. Puromycin was applied at three different concentrations shown in the graphs. NC: negative control; PC: positive control. Data was shown mean ± SD. n = 3 biological replicates.

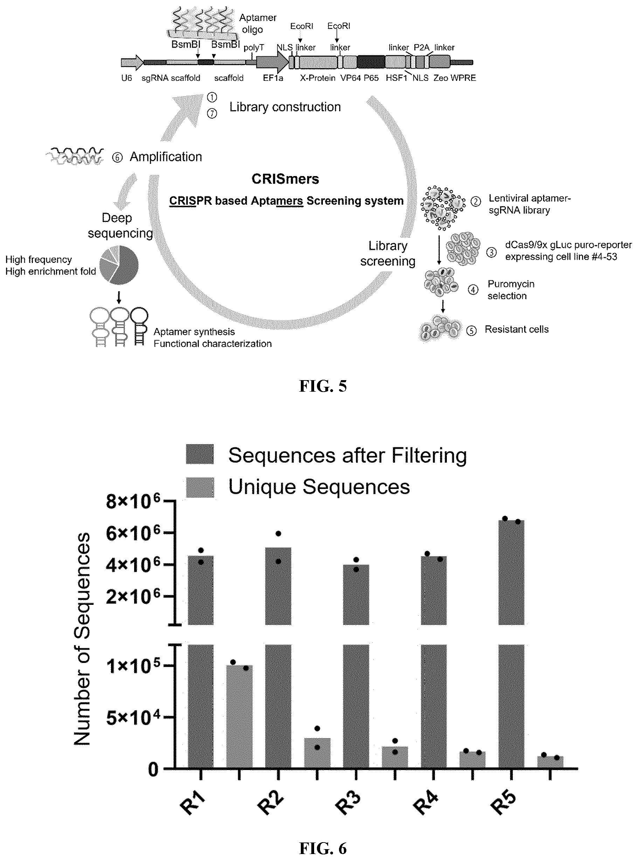

**Figure 5****.** Schematic illustration of the CRISmers RNA aptamer screening system based on CRISPR/Cas9.
**Figure 6****.** Deep sequencing analyses of harvested aptamer sequences using CRISmers against the RBD area of spike protein of SARS-CoV-2 wild type virus. R: Selection rounds. Filtered sequences: incorrect sequences were filtered out. Unique sequences: single/unique sequences.

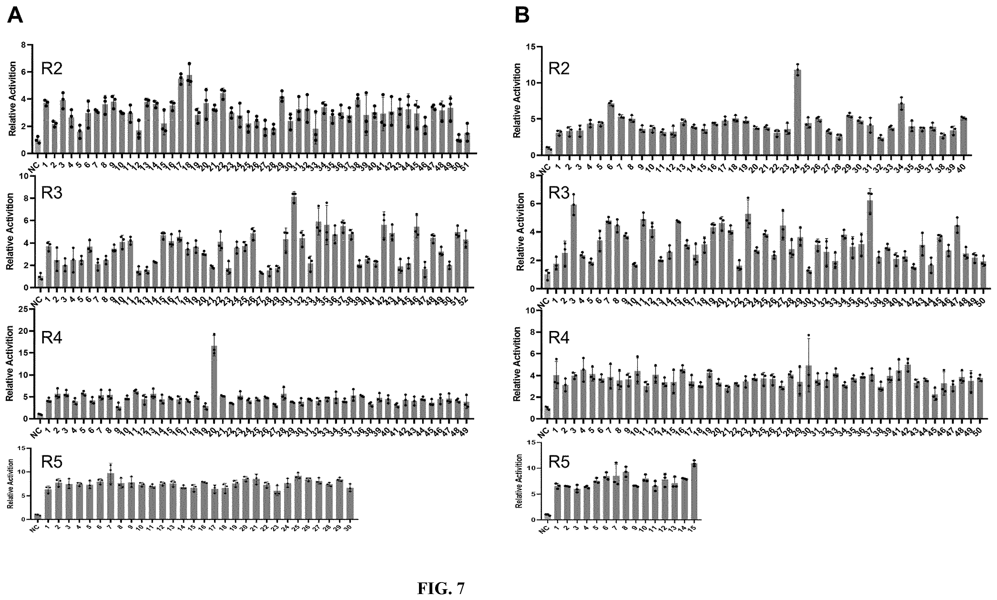

**Figure 7****.** Secondary screens using luciferase reporter assay. The top 0.1% frequency sequences and the top 15 high enrichment-fold sequences derived from deep sequencing of aptamer amplicons harvested from round 2 (R2), R3, R4, and R5 were screened. Two parallel CRISmers primary screens were conducted (presented separately in A and B). Data was shown as mean ± SD. n = 3 biological replicates.

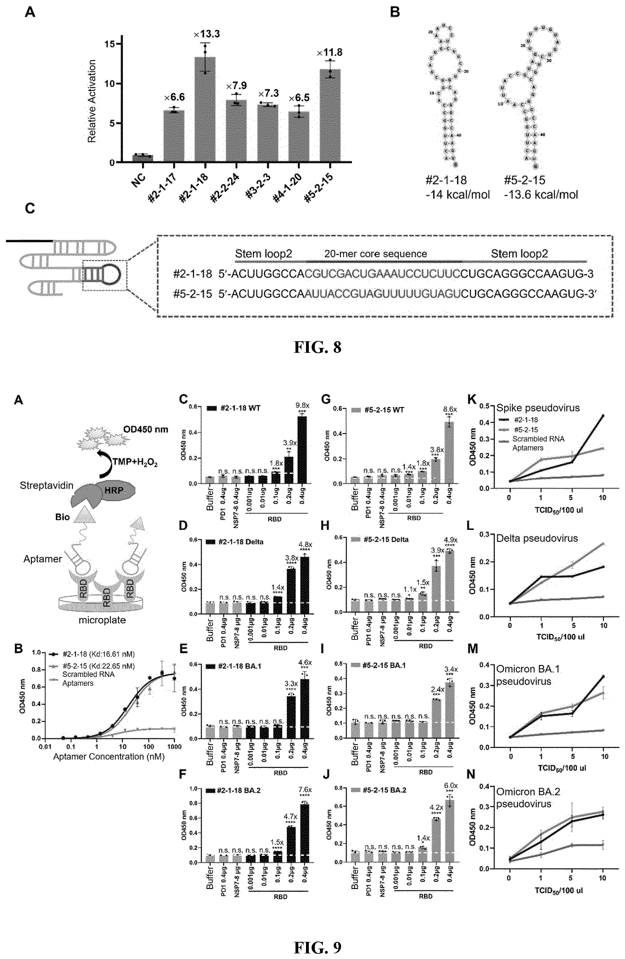

**Figure 8****.** CRISmers screening hits against the RBD area of spike protein of SARS-CoV-2 wild type virus. (A) Results of a luciferase reporter assay for parallel comparison of aptamer hits showing the highest activity from the secondary screen (Figure 7). (B) illustration of the sequences of two aptamers (#2-1-18 and #5-2-15) showing the highest fold of activation in the luciferase assay in Figure 7A. (C) The secondary structures and the free energy of the two aptamers predicted by Mfold webserver. Data show mean ± SD. n = 3 biological replicates.
**Figure 9****.** Examination of aptamer leads for detecting SARS-CoV-2. (A) A schematic depicting the enzyme-linked oligonucleotide assay (ELONA). (B) Examination of aptamers for their dose dependent activity to a fixed concentration of 250 ng of the RBD of SARS-CoV-2 Spike protein. A scrambled RNA aptamer was used as a negative control. Examination of binding activity to RBD recombinant protein of the original SARS-CoV-2 (C and G), Delta (D and H), Omicron BA.1 (E and I), and Omicron BA.2 (F and J) using 100 nM aptamers. Recombinant protein PD-1 extracellular domain and SARS-CoV-2 NSP7-8 complex were used as negative controls for specificity. Examination of binding activity to the original SARS-CoV-2 pseudovirus (K), Delta (L), and Omicron BA.1 and BA.2 (M, N) using 100 nM aptamers. A negative control RNA aptamer was used as a negative control for specificity. TCID50: 50% tissue culture infective dose. Data show mean ± SD. n = 3 biological replicates. **p* < 0.05, ***p* < 0.01, ****p <* 0.001, *****p <* 0.0001, two-tailed t-tests. n.s., no significant difference.

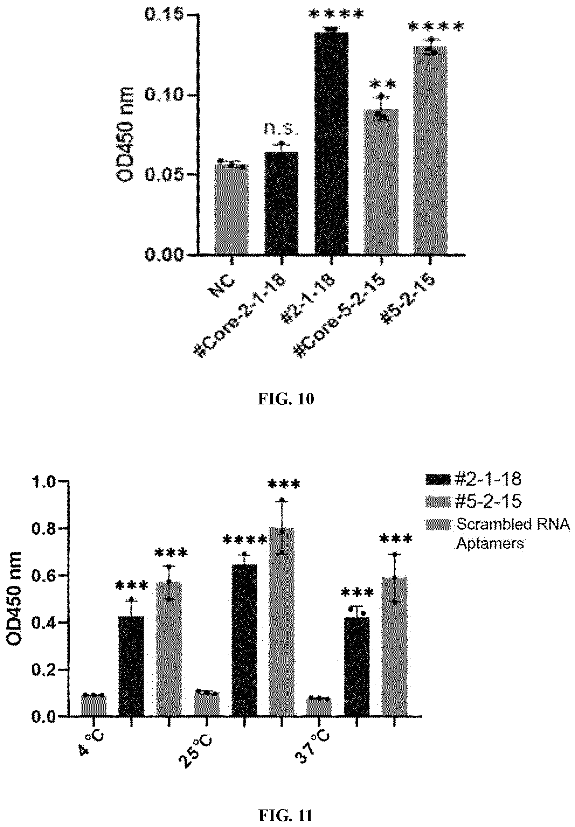

**Figure 10****.** Evaluation of the effect of constant sequences at both ends on the activity of RNA aptamers (ELONA). The binding activities to RBD protein were detected by ELONA for middle sequences only #Core-2-1-18 and #Core-5-2-15 and complete sequences #2-1-18 and # 5-2-15with constant sequence at both ends. A scrambled RNA aptamer was used as a negative control. Data show mean ± SD. n = 3 biological replicates. ***p <* 0.01, *****p <* 0.0001, two-tailed t-tests. n.s., no significant difference.
**Figure 11****.** Binding activities of RNA aptamers at different temperatures. The binding activities to RBD protein of RNA aptamers were detected by ELONA at 4 °C, 25 °C, and 37 °C. A scrambled RNA aptamer was used as a negative control. Data show mean ± SD. n = 3 biological replicates. ***p <0.001, ****p < 0.0001, two-tailed t-tests.

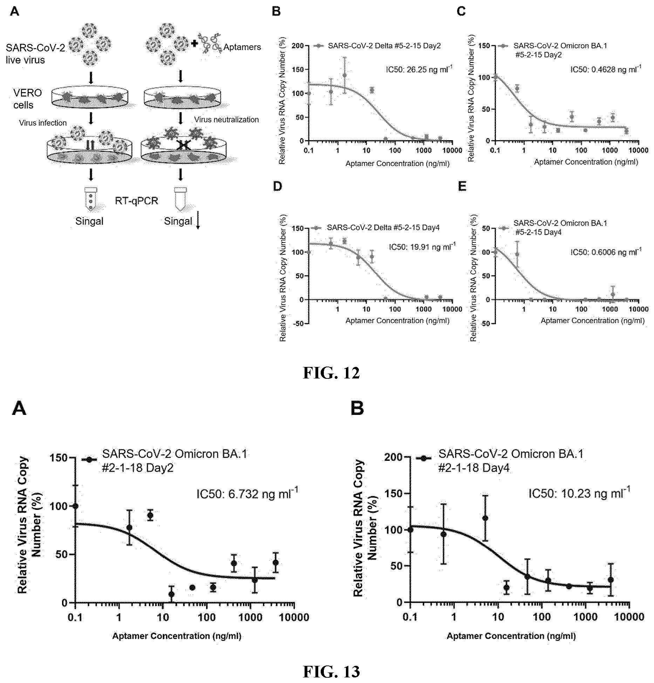

**Figure 12****.** Neutralizing activity of RNA aptamer #5-2-15 against live SARS-CoV-2 variants of concern. (A) A cartoon showing the working principle of SARS-CoV-2 live virus neuralization assay. Quantification of relative virus RNA copy number 2 days post infection to assess aptamer #5-2-15 for their activity against the infectivity of SARS-CoV-2 Delta (B) and SARS-CoV-2 Omicron BA.1(C). Quantification of relative virus RNA copy number on day 4 after infection to assess aptamer #5-2-15 for their activity against the infectivity of SARS-CoV-2 Delta (D) and SARS-CoV-2 Omicron BA.1(E). Readouts from negative controls served as 100% for normalization. Test samples were cell supernatants harvested 2 and 4 days post virus infection, respectively. IC50s were indicated. Data show mean ± SD. n = 3 biological replicates.
**Figure 13****.** Neutralizing activity of RNA aptamer #2-1-18 against live SARS-CoV-2 Omicron BA.1. Quantification of relative virus RNA copy number to assess aptamer #2-1-18 for their activity against the infectivity of SARS-CoV-2 Omicron BA.1 2 days (A) and 4 days (B) post infection. Readouts from negative controls served as 100% for normalization. Test samples were cell supernatants harvested 2 days post virus infection. IC50s were indicated. Data show mean ± SD. n = 3 biological replicates.

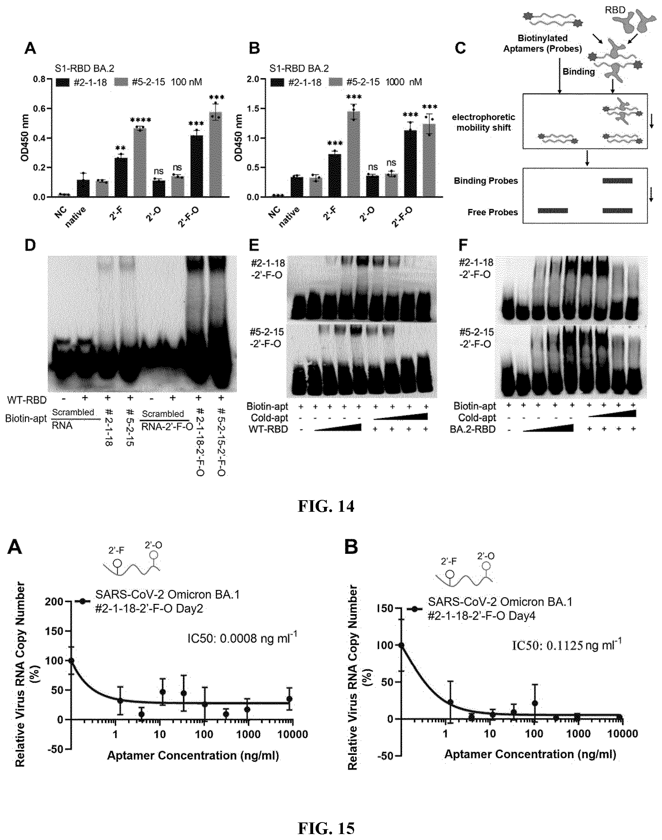

**Figure 14****.** Binding activity and specificity of modified aptamers. (A, B) The binding activities of unmodified and modified aptamers against a recombinant RBD of SARS-CoV-2 Omicron BA.2 spike protein were detected by ELONA. The concentrations of the aptamers were 100 nM (A) and 1,000 nM (B). Native: unmodified RNA aptamers. 2'-F: 2' position of pyrimidines with fluorin modification. 2'-O: 2' position of purines with 2'-O-methyl modification. 2'-F-O: 2' position of pyrimidines with fluorin modification and purines with 2'-O-methyl modification. (C) Schematic illustration of the protocol of electrophoretic mobility shift assay (EMSA). (D) The binding activities of unmodified and modified aptamers against a recombinant RBD of SARS-CoV-2 spike protein were detected by EMSA in parallel. (E, F) Results of dose-dependent binding activities (E) and dose-dependent competitive binding activities (F) of modified aptamers against a recombinant RBD of SARS-CoV-2 wild-type virus spike protein and a recombinant RBD of SARS-CoV-2 Omicron BA.2 spike protein, as determined by EMSA. A scrambled RNA aptamer was used as a negative control. Biotin-apt: Biotinylated aptamers. Cold-apt: unmodified aptamers. Data show mean ± SD. n = 3 biological replicates. ***p <* 0.01, ****p <* 0.001, *****p* < 0.0001, two-tailed t-tests. n.s., no significant difference.
**Figure 15****.** Neutralizing activity of modified RNA aptamer #2-1-18-2'-F-O against live SARS-CoV-2 Omicron BA.1. Quantification of relative virus RNA copy number to assess aptamer #2-1-18-2'-F-O for its neutralizing activity against SARS-CoV-2 Omicron BA.1 variant 2 days (A) and 4 days (B) post infection. Readouts from negative controls served as 100% for normalization. Test samples were cell supernatants harvested 2 days and 4 days post virus infection, respectively. IC50s were indicated. Data show mean ± SD. n = 3 biological replicates.

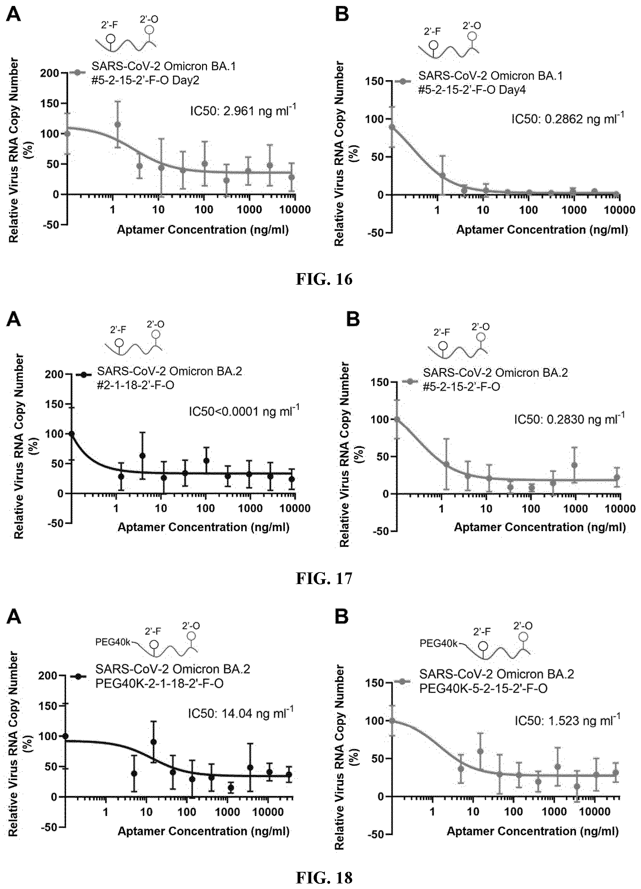

**Figure 16****.** Neutralizing activity of modified RNA aptamer #5-2-15-2'-F-O against live SARS-CoV-2 Omicron BA.1. Quantification of relative virus RNA copy number to assess aptamer #5-2-15-2'-F-O for its neutralizing activity against SARS-CoV-2 Omicron BA.1 variant 2 days (A) and 4 days (B) post infection. Readouts from negative controls served as 100% for normalization. Test samples were cell supernatants harvested 2 days and 4 days post virus infection, respectively. IC50s were indicated. Data show mean ± SD. n = 3 biological replicates.
**Figure 17****.** Neutralizing activity of modified RNA aptamers #2-1-18-2'-F-O and #5-2-15-2'-F-O against live SARS-CoV-2 Omicron BA.2 variant. Quantification of relative virus RNA copy number to assess aptamer #2-1-18-2'-F-O (A) and #5-2-15-2'-F-O (B) for their neutralizing activities against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.
**Figure 18****.** Neutralizing activity of aptamers with 5'-40 kDa PEG and 2'-F-O modifications (PEG40K-2-1-18-2'-F-O and PEG40K-5-2-15-2'-F-O) against live SARS-CoV-2 Omicron BA.2 variant. Quantification of relative virus RNA copy number to assess aptamers PEG40K-2-1-18-2'-F-O (A) and PEG40K-5-2-15-2'-F-O (B) for their neutralizing activities against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.

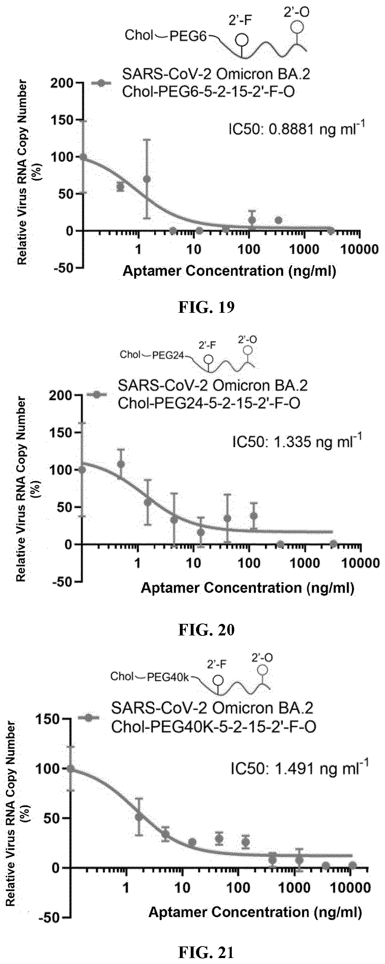

**Figure 19****.** Neutralizing activity of aptamer with 5'-cholesterol-PEG6 and 2'-F-O modifications (chol-PEG6-5-2-15-2'-F-O) against live SARS-CoV-2 Omicron BA.2 variant. Quantification of relative virus RNA copy number to assess aptamer chol-PEG6-5-2-15-2'-F-O for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.
**Figure 20****.** Neutralizing activity of aptamer with 5'-cholesterol-PEG24 and 2'-F-O modifications (chol-PEG24-5-2-15-2'-F-O) against live SARS-CoV-2 Omicron BA.2 variant. Quantification of relative virus RNA copy number to assess aptamer chol-PEG24-5-2-15-2'-F-O for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.
**Figure 21****.** Neutralizing activity of aptamer with 5'-cholesterol-40 kDa PEG and 2'-F-O modifications (chol-PEG40K-5-2-15-2'-F-O) against live SARS-CoV-2 Omicron BA.2 variant. Quantification of relative virus RNA copy number to assess aptamer chol-PEG40K-5-2-15-2'-F-O for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.

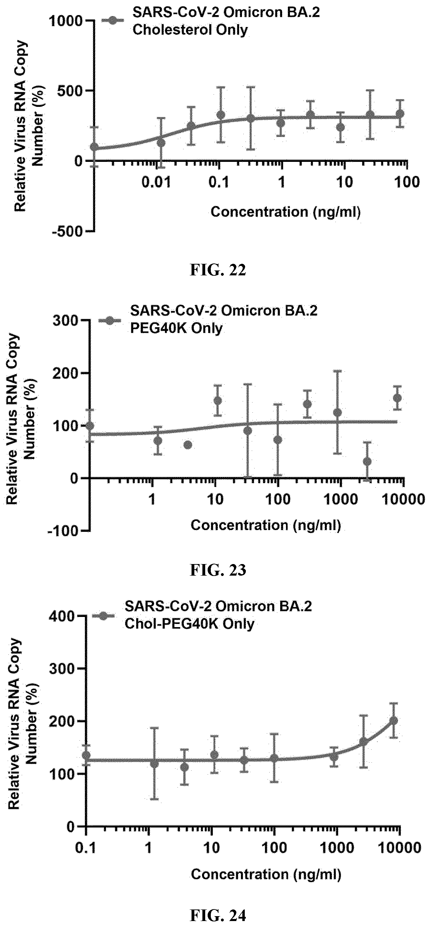

**Figure 22****.** Neutralizing activity of cholesterol against live SARS-CoV-2 Omicron BA.2 variant. Quantification of relative virus RNA copy number to assess cholesterol for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.
**Figure 23****.** Neutralizing activity of 40kDa PEG against live SARS-CoV-2 Omicron BA.2 variant. Quantification of relative virus RNA copy number to assess 40kDa PEG for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.
**Figure 24****.** Neutralizing activity of cholesterol-40kDa PEG against live SARS-CoV-2 Omicron BA.2 variant. Quantification of relative virus RNA copy number to assess cholesterol-40kDa PEG for its neutralizing activity against SARS-CoV-2 Omicron BA.2 variant. Readouts from negative controls served as 100% for normalization. Test samples were cell supernatants harvested 2 days post virus infection. IC50s were indicated. Data show mean ± SD. n = 3 biological replicates.

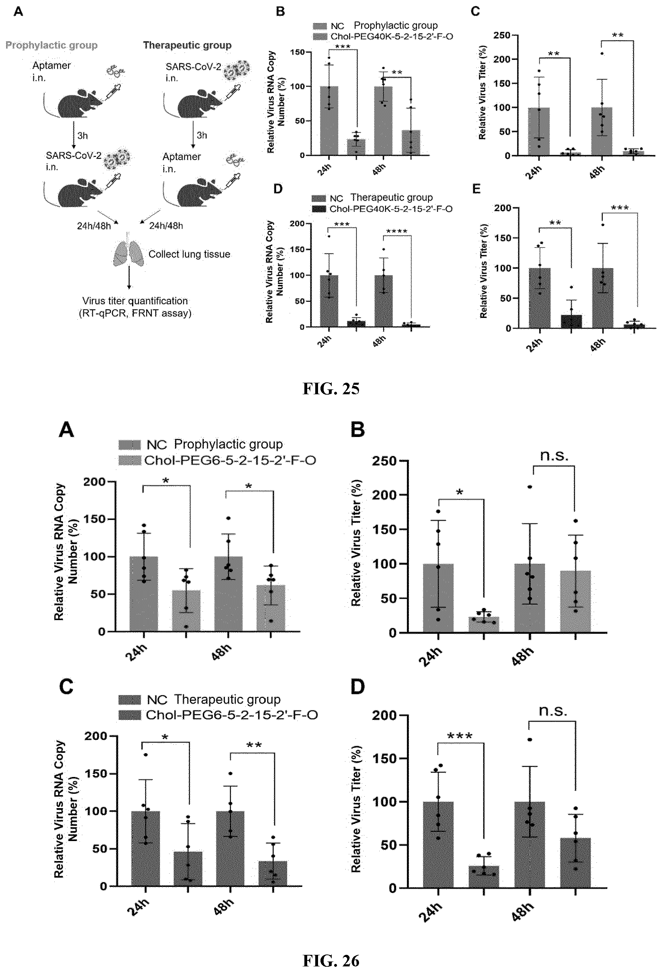

**Figure 25****.** Animal experiment of chol-PEG40K-5-2-15-2'-F-O. (A) A schematic depicting the animal experiment to examine the prophylactic and therapeutic effect of chol-PEG40K-5-2-15-2'-F-O against live SARS-CoV-2 Omicron BA.2 variant *in vivo*. (B) Quantification of relative RNA copy number of SARS-CoV-2 Omicron BA.2 variant in lung tissue by RT-qPCR in an experiment to assess the prophylactic effect. (C) Quantification of virus titer of SARS-CoV-2 Omicron BA.2 variant in lung tissue by FRNT in an experiment to assess the prophylactic effect. (D) Quantification of relative RNA copy number of SARS-CoV-2 Omicron BA.2 variant in lung tissue by RT-qPCR in an experiment to assess the therapeutic effect. (E) Quantification of virus titer of SARS-CoV-2 Omicron BA.2 variant in lung tissue by FRNT in an experiment to assess the therapeutic effect. FRNT assay: focus reduction neutralization test. Readouts from negative controls served as 100% for normalization. Data show mean ± SD. n = 3 biological replicates. **p < 0.01, ***p < 0.001, ****p < 0.0001, two-tailed t-tests.
**Figure 26****.** Animal experiment of chol-PEG6-5-2-15-2'-F-O. (A) Quantification of relative RNA copy number of SARS-CoV-2 Omicron BA.2 variant in lung tissue by RT-qPCR in an experiment to assess the prophylactic effect. (B) Quantification of virus titer of SARS-CoV-2 Omicron BA.2 variant in lung tissue by FRNT in an experiment to assess the prophylactic effect. (C) Quantification of relative RNA copy number of SARS-CoV-2 Omicron BA.2 variant in lung tissue by RT-qPCR in an experiment to assess the therapeutic effect. (D) Quantification of virus titer of SARS-CoV-2 Omicron BA.2 variant in lung tissue by FRNT in an experiment to assess the therapeutic effect. FRNT assay: focus reduction neutralization test. Readouts from negative controls served as 100% for normalization. Data show mean ± SD. n = 3 biological replicates. **p < 0.01, ***p < 0.001, ****p < 0.0001, two-tailed t-tests.

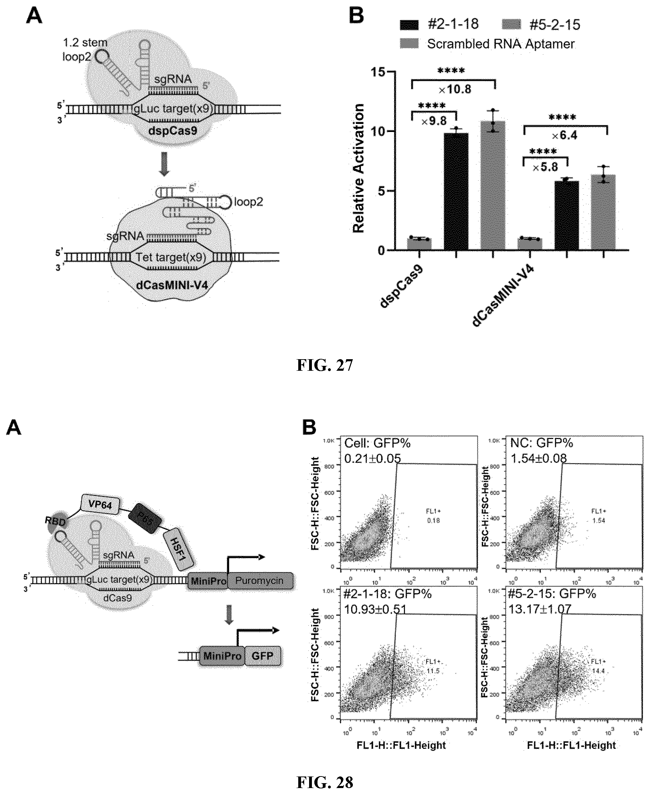

**Figure 27****.** Validation of CRISmers in different CRISPR/Cas systems. (A) schematic illustration showing dCasMINI-V4, an evolved version of Un1Cas12f1 CRISPR/Cas system, was used in place of CRISPR/Cas9 in CRISmers to evaluate its adaptability. The aptamer sequence was inserted into the loop 2 of dCasMINI-V4 sgRNA scaffold. (B) The firefly-luciferase reporter assay was used to examine activation. A scrambled RNA aptamer was used as a negative control. Data show mean ± SD. n = 3 biological replicates. ***p < 0.001, ****p < 0.0001, two-tailed t-tests.
**Figure 28****.** Validation of CRISmers with different selection markers. (A) Switching the CRISmers selection marker from puromycin report gene to GFP. The binding affinity of an aptamer to a target protein was translated to the expression capability of GFP. (B) Flow Cytometry was used to detect the activation efficiency of GFP mediated by aptamers #2-1-18 and #5-2-15. Cell: blank cell group. NC: negative control. GFP%: percentage of GFP positive cells.

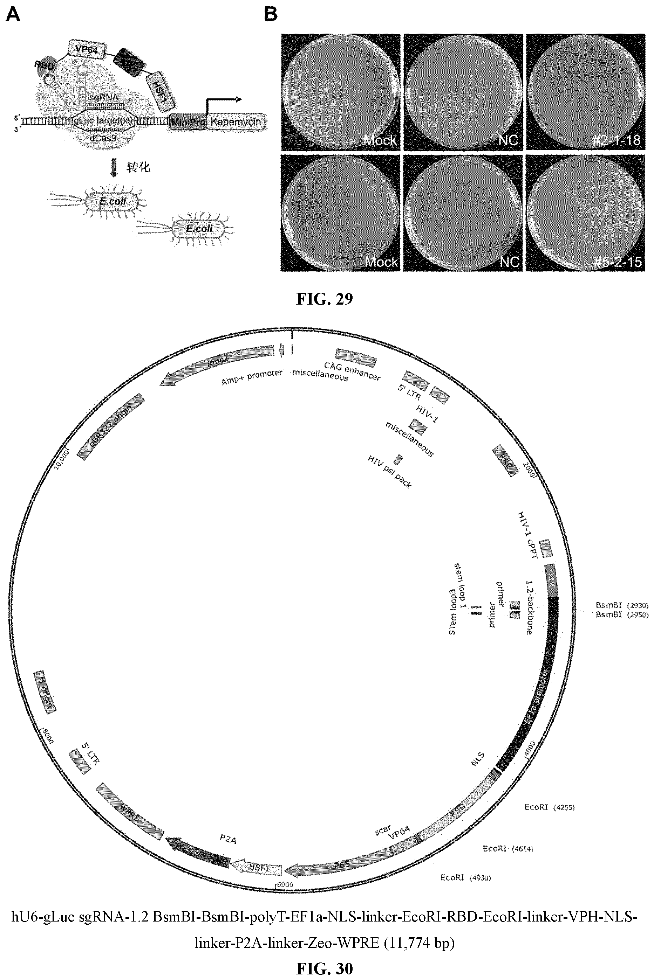

**Figure 29****.** Validation of CRISmers in different host species. (A) Switching host cells from human HEK293T to bacteria *E. coli.* Kanamycin was used for selection. (B) Performance of CRISmers in *E.coli.*
**Figure 30****.** Schematic view of a customizable lentivirus vector for use in screening in an eukaryotic HEK293T-dCas9 cell line (phU6-gLuc sgRNA-1.2 BsmBI-BsmBI-polyT-EF1a-NLS-linker-EcoRI-EcoRI-linker-VP64-P65-HSF1-NLS-linker-P2A-linker-Zeo-WPRE).

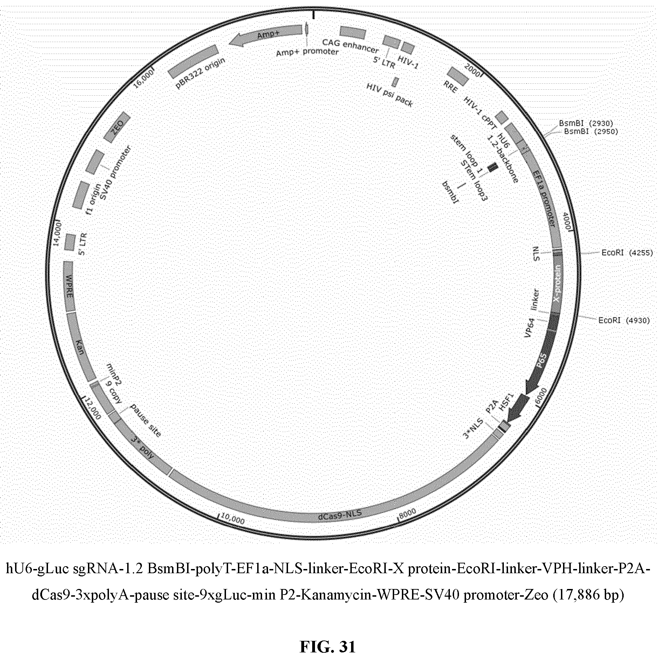

**Figure 31****.** Schematic view of a customizable plasmid vector for use in screening in a prokaryotic *E. coli* cell line (phU6-gLuc sgRNA-1.2 BsmBI-BsmBI-polyT-EF1a-NLS-linker-EcoRI-X-protein-EcoRI-linker-VP64-P65-HSF1-NLS-linker-P2A-dCas9-WPRE-SV40 promoter-Zeo).

### Detailed Description of the Disclosure

### Definition

The term "a" or "an" refers one or more of the entities. For example, "an exosome" is understood to be one or more exosomes. Therefore, the terms "a/an", "one or more" and "at least one" is used interchangeably in this disclosure.

The terms "subject", "patient" or "individual" as used herein refers to any subject that is desirable for diagnosis, prognosis or treatment, especially to mammal subject. A mammal includes humans, domestic animals, farm animals, zoo animals, competitive animals or pets, such as dogs, cats, pigs, rabbits, rats, mice, horses, cows, dairy cows and etc. The subject in this disclosure is preferably a human being.

As used herein, the term "treatment" refers to therapeutic and prophylactic measures that prevent or slow the occurrence of undesired physiological alterations or disorders in a subject, such as upper respiratory and gastrointestinal symptoms including viral infection, fever, dry cough, and fatigue. Beneficial or desirable clinical outcomes include, but are not limited to, reduction or elimination of viral load, alleviation of symptoms, diminution of disease level, stabilization of disease status (i.e., absence of deterioration), delay or deceleration of disease progression, mitigation or amelioration of disease condition, and partial or complete cure of the disease, irrespective of whether such effects are detectable. "Treatment" may also denote prolongation of survival duration compared to absence of treatment. Subjects requiring treatment encompass those already afflicted with the disease or disorder, those potentially susceptible to the disease or disorder, and those requiring prophylaxis against the disease or disorder.

In the present disclosure, the term "patient in need of treatment" or "subject in need of treatment" is defined to include subjects benefiting from administration of the polypeptide or composition thereof according to the invention for detection, diagnostic, and/or therapeutic applications, including but not limited to mammalian subjects.

As used herein, the term "therapeutically effective amount" or "effective amount" is defined as a quantity of the pharmaceutical agent or pharmaceutical composition of the present invention that, when administered alone or in combination with additional therapeutic agents to a cell, tissue, or treated subject, effectively prevents or mitigates the progression of a disease or disorder requiring the treatment. The therapeutically effective dose further refers to an amount of said compound sufficient to induce amelioration of symptoms, including but not limited to treatment, cure, prevention, or retardation of an associated medical condition, or enhancement of therapeutic, curative, preventive, or mitigatory rates relative to said pathology. When administering an active ingredient as a monotherapy, the therapeutically effective amount refers to said sole active ingredient. When administered in combination, the therapeutically effective amount denotes the combined quantity of active ingredients producing therapeutic efficacy, whether administered in combination, sequentially, or simultaneously. The therapeutically effective amount shall achieve symptom alleviation by: typically at least 10%; ordinarily at least 20%; preferably at least approximately 30%; more preferably at least 40%; and most preferably at least 50%.

In the present invention, the term "about" is defined to encompass numerical values within an acceptable error range of a specified value as determined by a person of ordinary skill in the art, said range being partially dependent on the measurement methodology employed (i.e., limitations of the measurement system). For instance, in certain embodiments of the art, "about" may denote within one standard deviation or exceeding one standard deviation. Alternatively, "about" or "substantially comprising" may signify a variation of up to 20%. Furthermore, with respect to biological systems or processes, the term may denote a variation of up to one order of magnitude or up to fivefold of the numerical value. Unless otherwise specified in the present disclosure, when specific numerical values appear in this application and claims, the meanings of "about" or "substantially comprising" shall be presumed to signify values within the acceptable error range of said specified value.

As used herein, the term "complementary" is defined as the capacity for precise base pairing between two nucleobases of oligomeric compounds. For example, if a nucleobase at a given position of an oligonucleotide (oligomeric compound) is capable of forming hydrogen bonds with a nucleobase at a corresponding position of a target nucleic acid, said positions in the oligonucleotide and target nucleic acid are designated as complementary positions, and said target nucleic acid is DNA, RNA, or an oligonucleotide molecule. Mutual complementarity between an oligonucleotide and another DNA, RNA, or oligonucleotide molecule exists when a sufficient number of complementary positions in each molecule are occupied by nucleobases capable of forming inter-base hydrogen bonds. The term "complementary" therefore denotes a sufficient degree of precise base pairing or complementarity by sufficient quantity of nucleobase pairs that enables them to establish stable and specific binding between the oligonucleotide and target nucleic acid.

It is understood that the constant sequences at both ends of the nucleic acid aptamers do not require 100% complementarity for specific hybridization forming a stem structure. Furthermore, oligonucleotides may hybridize across one or more contiguous segments such that intervening or adjacent segments remain unpaired in hybridization events (e.g., loop structures or hairpin configurations). Preferably, the constant sequences at both termini of the nucleic acid aptamer according to the invention exhibit sequence complementarity of at least 70%, at least 75%, at least 80%, or at least 85%; more preferably at least 90%; and even more preferably at least 95% or at least 99%. For instance, 18 out of 20 nucleobases in a 5'-terminal constant sequence complementary to a 3'-terminal constant sequence represents 90% complementarity. In this embodiment, the remaining non-complementary nucleobases may be clustered with complementary nucleobases or interspersed therebetween, without requiring adjacency to each other or to complementary nucleobases. Thus, an antisense oligomer with 18 nucleobases in length containing 4 non-complementary nucleobases flanked by two regions of perfect complementarity to a target nucleic acid demonstrates 77.8% overall complementarity, thereby falling within the scope of the present invention. The percentage complementarity between 5'-terminal and 3'-terminal constant sequences may be routinely determined using the Basic Local Alignment Search Tool (BLAST) algorithm and PowerBLAST program, both well-established in the art.

As used herein, the terms "gLuc sequence" and "Tet sequence" refer to exogenous artificial sequences exogenous to the human genome, characterized by enhanced targeting capability while mitigating off-target effects associated with endogenous genomic loci engagement.

As used herein, the term "linker" is defined as a short nucleic acid construct comprising two or more nucleotide units, which may be identical or distinct, selected from the group consisting of adenine (A), guanine (G), cytosine (C), thymine (T), and uracil (U).

As used herein, the term "promoter" is defined as a DNA sequence recognized, bound, and transcriptionally activated by RNA polymerase, comprising conserved sequences requisite for polymerase-specific binding and transcription initiation, predominantly positioned upstream of the transcriptional start site of structural genes, wherein said promoter sequence is not itself transcribed. The "basal promoter" or "minimal promoter" refers to a promoter configuration consisting exclusively of fundamental transcriptional initiation elements, including but not limited to TATA-box motifs and/or initiator codons, which operationalize transcription initiation upon interaction with appropriate transcription factors. In some embodiments, the first promoter is selected from the group consisting of U6, 7SK, and H1 promoters. In some embodiments, the second promoter comprises the EF1a promoter. In some embodiments, the basal promoter contained in the selection marker is selected from the group consisting of: mini-promoter-1 (SEQ ID NO: 28), mini-promoter-2 (SEQ ID NO: 27), mini-TK promoter (SEQ ID NO: 29), mini-CMV promoter (SEQ ID NO: 30), and Crystallin basal promoter (SEQ ID NO: 31).

*CRISPR*/*Cas.* As used herein, the term "CRISPR-Cas system" refers to an adaptive immune defense mechanism originating from bacteria and archaea, configured to protect host organisms against invasion by exogenous viral and plasmid-derived nucleic acids. Within this system, CRISPR sequences are transcribed and processed into non-coding CRISPR RNAs (crRNAs) that directly target DNA substrates rather than RNA. The guide RNA (gRNA), alternatively termed single guide RNA (sgRNA), functions in a post-transcriptional modification process termed RNA editing within kinetoplastid organisms, representing a distinct class of compact non-coding RNAs. CRISPR-associated proteins (Cas proteins) are endonucleases capable of recognizing and cleaving sequence-complementary DNA strands through RNA-guided targeting mediated by spacer sequences embedded within CRISPR arrays. The term "deactivated Cas" (dCas) denotes Cas proteins rendered catalytically inert via point mutations while retaining DNA-binding capability. dCas proteins may be functionally fused with transcriptional repression domains (TRDs) or transcriptional activation domains (TADs). dCas proteins may also effector proteins including transcriptional activators, repressors, and epiregulators, Such chimeric constructs enable gene-specific CRISPR-mediated activation (CRISPRa), interference (CRISPRi), and epigenomic modifications. In some embodiments, the dCas protein is dCas9. In some embodiments, the dCas9 protein is dCasMINI-V4.

CRISPR/Cas9 system is a molecular architecture wherein CRISPR-derived RNAs (crRNAs) direct Cas9-mediated induction of double-strand breaks (DSBs) in target DNA. Adjacent to CRISPR arrays reside trans-activating CRISPR RNAs (tracrRNAs), which exhibit complementarity to repeat sequences and undergo RNase III-mediated cleavage in the presence of Cas9, thereby generating mature crRNA complexes. In 2012, Jennifer et al. pioneered the covalent fusion of tracrRNA and crRNA into a single-guide RNA (sgRNA) chimera, demonstrating for the first time in vitro the capability of *Streptococcus pyogenes*-derived CRISPR/Cas9 (spCas9 system) to execute sgRNA-directed Cas9-mediated DNA cleavage.

The CRISPR/Cas9 system achieves sequence-specific targeting through sgRNA-DNA base-pair complementarity, guiding nuclease-active Cas9 protein to recognize and cleave DNA sequences complementary to the sgRNA, provided said sequences are flanked by a 3'-terminal protospacer adjacent motif (PAM) conforming to the NGG consensus. This cleavage event triggers endogenous DNA repair mechanisms, including non-homologous end joining (NHEJ) and homology-directed repair (HDR), thereby enabling programmable genome editing events.

"dCas9" refers to a catalytically inactivated Cas9 variant engineered through site-directed mutagenesis of nuclease domains, for example specifically comprising H840A substitution in the HNH domain and D10A substitution in the RuvC domain. This deactivated/dead Cas9 (dCas9) retains precision targeting capability guided by gRNA for sequence-specific DNA binding while lacking endo-nucleolytic cleavage activity. The dCas9 complex is capable of recruiting effector moieties, including transcriptional repressors, or activator domains, to genomic loci encompassing promoter regions, regulatory elements, or coding sequences. This architecture enables site-specific gene regulation without DNA damage.

As used herein, the term "CasMINI" refers to an engineered variant of Un1Cas12f1 optimized for reduced molecular dimensions, representing a next-generation compact genome-editing system with substantially smaller size compared to conventional Cas9 nucleases. In the present invention, we employed a dead version of CasMINI designated as dUn1Cas12f1 (dCasMINI-V4, where V4 denotes Version 4 of the deactivated construct), which retains sequence-specific targeting capability for genome binding while being devoid of endo-nucleolytic cleavage activity.

*The RBD region of SARS-CoV-2 S1 protein.* Global emergence and sustained proliferation of Severe Acute Respiratory Syndrome Coronavirus 2 (SARS-CoV-2)-induced coronavirus disease 2019, herein designated COVID-19, which has posed grave threats to global public health and human security since its initial outbreak. Amidst persistent viral evolution, SARS-CoV-2 has diverged from its ancestral wild-type strain to generate a spectrum of phylogenetically distinct variant strains, with particular epidemiological significance attributed to Delta and Omicron variants due to their enhanced transmissibility profiles.

"SARS-CoV-2" is a member of the genus Betacoronavirus within the family Coronaviridae, characterized as an enveloped, single-stranded positive-sense RNA virus. The SARS-CoV-2 genome comprises a 5'-capped and 3'-polyadenylated RNA structure encoding 29 proteins, including 25 non-structural proteins (Nsps) and accessory proteins and 4 structural proteins. The non-structural proteins mediate critical functions in viral RNA replication and immune evasion mechanisms, while accessory proteins facilitate viral infection, survival, and intercellular spreading. Structural proteins orchestrate virion assembly and maturation. Genomically, the 5'-proximal two-thirds encode two open reading frames (ORF1a and ORF1b) that translate into replicase polyproteins. The 3'-terminal one-third contains multiple ORFs, four of which encode the structural proteins, i.e., spike glycoprotein (S), membrane protein (M), envelope protein (E), and nucleocapsid protein (N), and the others of which encode multiple accessory proteins.

"SARS-CoV-2 S protein" is a type I viral fusion protein comprising two functional subunits: S1 and S2. Coronaviral entry into host cells is mediated by the S protein, wherein the S1 subunit facilitates receptor binding to host cell angiotensin-converting enzyme 2 (ACE2) via its receptor-binding domain (RBD), while the S2 subunit governs viral-cell membrane fusion. Therefore, the S1 subunit or its RBD region constitutes critical therapeutic targets for interrupting viral-host receptor (hACE2) interactions, serving as focal points for COVID-19 vaccine development, neutralizing antibody engineering, and SARS-CoV-2 diagnostic assay design. In the present invention, RNA aptamers selected through the nucleic acid aptamer screening system demonstrate sensitive detection of S protein and/or its RBD region and effective blockade of RBD-hACE2 binding interactions.

*Nucleic Acid Aptamers.* As used herein, the term "nucleic acid aptamers" refers to short single-stranded DNA (ssDNA) or RNA oligonucleotide molecules that fold into complicate and distinctive three-dimensional conformations, enabling high-specificity and high-affinity binding to target protein molecules. Compared to ssDNA aptamers, RNA aptamers exhibit enhanced structural plasticity due to their capacity to form more complex spatial architectures.

Nucleic acid aptamers are prone to form complementary base pairings that confer distinct three-dimensional structure. These aptamers fold into secondary structural elements including but not limited to stems, loops, bulges, pseudoknots, G-quadruplexes, and kissing hairpins. The hierarchical assembly of these secondary structures generates unique three-dimensional conformational architectures that enable molecular recognition of target species. The binding affinity and specificity of aptamer-target interactions are critically governed by hydrophobic and electrostatic interactions, hydrogen bonding, Van der Waals forces, structural complementarity, and base stacking. Analogous to antibody-antigen binding paradigms, the formation of aptamer-target molecular complexes is driven by specific three-dimensional interface interactions.

In one aspect of the invention, provided is an RNA aptamer specifically binding to S1 protein of SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the SARS-CoV-2 virus is SARS-CoV-2 wild-type virus, or its Delta, Omicron BA.1 or BA.2 variants. The RNA aptamers targeting the SARS-CoV-2 S1 protein according to the present invention are postulated to demonstrate pan-variant applicability to both currently circulating and future-emergent SARS-CoV-2 variant strains, based on their broad-spectrum binding characteristics.

Experiments demonstrated that terminal constant sequences facilitate oligonucleotide synthesis and amplification while conferring significant enhancement to RNA aptamer binding affinity against target protein molecules. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9.

In addition, it is contemplated that a sequence having at least about 70%, at least about 75%, at least about 80%, at least about 85%, at least about 90%, at least about 95%, or at least about 97% sequence identity to any one of the nucleic acid sequences set forth in SEQ ID NO: 1 to SEQ ID NO: 9 are also within the scope of the present disclosure.

*Modified RNA Aptamers.* Chemical modifications of nucleic acid aptamers serve to enhance the stability of nucleic acid molecules, prolong their in vivo half-life, and mitigate immunogenic potential.

In one aspect, provided is a chemically modified RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7, and wherein the chemical modification is one or more modifications selected from a group consisting of fluorine substitution, O-methylation, PEGylation and cholesterol-PEGylation. In some embodiments, the PEGylation is selected from hexapolyethylene glycol to a PEG having a 40 kD molecule weight. In some embodiments, the chemical modification is one or more modifications selected from a group consisting of fluorine substitution, O-methylation, cholesterol-PEG6 modification, cholesterol-PEG24 modification, PEG 40kDa modification, and cholesterol-PEG40kDa modification. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the SARS-CoV-2 virus is SARS-CoV-2 wild-type virus, or its Delta, Omicron BA.1 or BA.2 variants.

In some embodiments, the chemical modification is fluorine substitution, wherein the hydroxyl group at 2'-position of the pyrimidine of the chemically modified RNA aptamer is substituted by fluorine.

In some embodiments, the chemical modification is O-methylation, wherein the hydroxyl group at 2'-position of the purine of the chemically modified RNA aptamer is substituted by O-methyl group.

In some embodiments, the chemical modification is fluorine substitution and O-methylation, wherein the hydroxyl group at 2'-position of the pyrimidine of the chemically modified RNA aptamer is substituted by fluorine and the hydroxyl group at 2'-position of the purine of the chemically modified RNA aptamer is substituted by O-methyl group.

In some embodiments, the chemical modification is a combination of fluorine substitution, O-methylation and conjugation of cholesterol-PEG6, wherein the hydroxyl group at 2'-position of the pyrimidine of the chemically modified RNA aptamer is substituted by fluorine, the hydroxyl group at 2'-position of the purine of the chemically modified RNA aptamer is substituted by O-methyl group and the 5'-end of the chemically modified RNA aptamer is conjugated with cholesterol-PEG6.

In some embodiments, the chemical modification is a combination of fluorine substitution, O-methylation and conjugation of cholesterol-PEG24, wherein the hydroxyl group at 2'-position of the pyrimidine of the chemically modified RNA aptamer is substituted by fluorine, the hydroxyl group at 2'-position of the purine of the chemically modified RNA aptamer is substituted by O-methyl group and the 5'-end of the chemically modified RNA aptamer is conjugated with cholesterol-PEG24.

In some embodiments, the chemical modification is a combination of fluorine substitution, O-methylation and conjugation of PEG 40kDa, wherein the hydroxyl group at 2'-position of the pyrimidine of the chemically modified RNA aptamer is substituted by fluorine, the hydroxyl group at 2'-position of the purine of the chemically modified RNA aptamer is substituted by O-methyl group and the 5'-end of the chemically modified RNA aptamer is conjugated with PEG 40kDa.

In some embodiments, the chemical modification is a combination of fluorine substitution, O-methylation and conjugation of cholesterol-PEG 40kDa, wherein the hydroxyl group at 2'-position of the pyrimidine of the chemically modified RNA aptamer is substituted by fluorine, the hydroxyl group at 2'-position of the purine of the chemically modified RNA aptamer is substituted by O-methyl group and the 5'-end of the chemically modified RNA aptamer is conjugated with cholesterol-PEG 40kDa.

*Identity.* As used herein, the terms "homology," "identity," or "similarity" refer to sequence correspondence between two polypeptides or two nucleic acid molecules. Homology is determined by comparing aligned sequence positions between the molecules, wherein sequences are aligned for comparative analysis. A position is deemed homologous when occupied by identical nucleotide bases or amino acid residues in both compared sequences. The degree of sequence homology represents a function of the number of matching positions shared by the sequences. The terms "unrelated" or "non-homologous" describe sequences sharing less than 40% identity (preferably less than 25% identity) with any sequence disclosed in the present invention.

As used herein, a polynucleotide or polynucleotide region (or polypeptide/polypeptide region) exhibiting a percentage sequence identity (e.g., 60%, 65%, 70%, 75%, 80%, 85%, 90%, 95%, 98%, or 99%) to another sequence signifies that said percentage of nucleotides (or amino acid residues) are identical when the sequences are comparatively aligned. Such alignment and percentage homology/sequence identity may be determined using software algorithms established in the art.

*Viral Vectors.* As used herein, the term "viral vector" is defined as a genetically engineered delivery vehicle that exploits the native molecular machinery of viral infection to transport exogenous genetic material into target cells. Viral vectors may alternatively be designated as vectors, vector virions, or vector particles. Exemplary viral vectors include, but are not limited to, retroviruses, adenoviruses, adeno-associated viruses (AAV), herpes simplex viruses (HSV), vaccinia viruses, baculoviruses, and lentiviruses.

Retroviral vectors may be derived from or derivable from any suitable retrovirus. A multiplicity of distinct retroviruses have been identified, with exemplary embodiments including, but not limited to, Murine Leukemia Virus (MLV), Human T-cell Leukemia Virus (HTLV), Mouse Mammary Tumor Virus (MMTV), Rous Sarcoma Virus (RSV), Fujinami Sarcoma Virus (FuSV), Moloney Murine Leukemia Virus (Mo-MLV), FBR Murine Osteosarcoma Virus (FBR MSV), Moloney Murine Sarcoma Virus (Mo-MSV), Abelson Murine Leukemia Virus (A-MLV), Avian Myelocytomatosis Virus-29 (MC29), and Avian Erythroblastosis Virus (AEV).

Adenoviruses are double-stranded linear DNA viruses that replicate without RNA intermediates. Adenoviruses are non-enveloped double-stranded DNA viruses capable of transducing a broad range of human and non-human cell types in vivo, ex vivo, and in vitro.

Adeno-associated virus (AAV), also known as adenovirus-associated virus, belongs to the genus *Dependoparvovirus* within the family *Parvoviridae.* It represents the simplest single-stranded DNA defective viruse discovered to date. Recombinant AAV vectors have been successfully employed for in vitro, ex vivo, and in vivo transduction of marker genes and genes implicated in human diseases. Certain AAV vectors have been engineered to efficiently accommodate large payloads (up to 8-9 kb).

Herpes simplex virus (HSV) is an enveloped double-stranded DNA virus that naturally infects neurons. It can accommodate large segments of exogenous DNA and has been adapted as a vector for gene delivery to neurons. Therapeutic applications of HSV require the use of attenuated strains engineered to prevent establishment of lytic cycles.

The viral vector of the present invention may be a vaccinia virus vector, such as MVA or NYVAC. It shall be understood that portions of the viral genome may remain intact following the insertion of recombinant genes. This implies the concept that the viral vector can retain the ability to infect cells and subsequently express additional genes that support its replication and potentially facilitate the lysis and death of infected cells.

Lentiviruses form part of the broader retrovirus group. They can be classified into primate and non-primate groups. Examples of primate lentiviruses include, but are not limited to, human immunodeficiency virus (HIV), the causative agent of acquired immunodeficiency syndrome (AIDS), and simian immunodeficiency virus (SIV). The non-primate lentivirus group comprises the prototype "lentivirus" visna/maedi virus (VMV), along with related caprine arthritis-encephalitis virus (CAEV), equine infectious anemia virus (EIAV), feline immunodeficiency virus (FIV), and bovine immunodeficiency virus (BIV).

*Non-Viral Vectors.* Non-viral vectors utilize the physicochemical properties of non-viral carrier materials to mediate gene transfer. Any suitable non-viral vector may be employed to introduce nucleic acid aptamers into target cells of a subject. Exemplary non-viral vectors include, but are not limited to, plasmids, liposomes, inorganic nanoparticles, and exosomes.

Plasmids are small circular DNA molecules that serve as the most commonly used and simplest vectors in genetic engineering. For use as expression vectors, they typically comprise three essential components: a genetic marker gene, a replication origin, and a gene of interest. Plasmids are found across all bacterial taxa as self-replicating DNA molecules independent of the bacterial chromosome. Examples of plasmid vectors include, but are not limited to, Escherichia coli plasmid vectors, Bacillus subtilis plasmid vectors, yeast plasmid vectors, Agrobacterium plasmid vectors, and cyanobacterial plasmid vectors.

Liposomes, artificially engineered hollow vesicles prepared from lipids such as lecithin and ceramide, exhibit a bilayer membrane structure. The composition of liposomes typically involves combinations of phospholipids (particularly those with high phase transition temperatures), often combined with sterols (especially cholesterol). Other phospholipids or lipids may also be utilized. The physical characteristics of liposomes depend on pH, ionic strength, and the presence of divalent cations. Transduction efficiency can be enhanced through the use of dioleoylphosphatidylethanolamine (DOPE) during transduction. High-efficiency liposomes are commercially available. Examples of liposomes include, but are not limited to, neutral liposomes, negatively charged liposomes, and positively charged liposomes.

Inorganic nanoparticles primarily function in therapeutic interventions by traversing cell membranes to transport pharmaceutical agents or biomolecules into organisms. Examples of inorganic nanoparticles applied in gene delivery include, but are not limited to, silicon, iron oxides, carbon nanotubes, calcium phosphate, metallic nanoparticles, quantum dots, and others.

Exosomes are small extracellular vesicles with an average diameter of 50-150 nm. They serve as mediators of intercellular communication. Typically, they comprise structural proteins alongside selected proteins, miRNAs, mRNAs, and long non-coding RNAs. RNAs contain short nucleotide sequences recognized by proteins that transport them into the cytoplasm and package them into exosomes. Exosomes transfer payloads from one cell to another. Upon entering recipient cells, the exosomal payload is released into the cytoplasm.

### Pharmaceutical Compositions and Pharmaceutical Kits

Pharmaceutical agents are substances used for the prevention, treatment, and diagnosis of diseases. Theoretically, the term encompasses any chemical substance capable of influencing the physiological functions of organs and tissues or modulating cellular metabolic activities within an organism.

In one aspect, provided is a pharmaceutical agent comprising an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

The term "pharmaceutical composition" as used herein refers to a pharmaceutical preparation and/or formulation for human use. The pharmaceutical composition comprises a suitable formulation that comprises the pharmaceutical agent as disclosed herein and a carrier, a stabilizer and/or an excipient.

Another aspect of the present invention provides a pharmaceutical composition comprising a pharmaceutical agent as disclosed herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

To prepare pharmaceutical compositions or sterile compositions, the active pharmaceutical agent is mixed with pharmaceutically acceptable carriers or excipients. Formulations of therapeutic and diagnostic agents, such as lyophilized powders, slurries, aqueous solutions, or suspensions, may be prepared by combining the active ingredient with physiologically acceptable carriers, excipients, or stabilizers.

Pharmaceutically acceptable excipients are well-known in the art. As used herein, "pharmaceutically acceptable excipients" refer to materials that, when combined with the active ingredient of the composition, preserve the biological activity of the ingredient and do not elicit disruptive reactions from the subject's immune system. These may include stabilizers, preservatives, salts, sugar complexes, or crystalline forms, among others. "Pharmaceutically acceptable" denotes molecules and components that do not induce allergic reactions or similar undesirable responses when administered to humans. Methods for preparing aqueous compositions containing the active ingredient as a functional component are established in the art. Typically, such compositions are formulated as injectable or sprayable preparations, such as liquid solutions or suspensions; they may also be prepared in solid forms suitable for reconstitution into solutions or suspensions prior to injection or spray.

The pharmaceutical agents or compositions of the present invention may be used individually or in combination with one another. Accordingly, the invention provides a pharmaceutical kit to facilitate the aforementioned combination therapy, comprising two or more of the pharmaceutical agents or compositions stored independently. In some embodiments, the subject may be administered two or more of the pharmaceutical agents or compositions simultaneously. In other embodiments, the subject may receive two or more of the pharmaceutical agents or compositions separately.

### Screening, Treatment and/or Prevention Methods

In another aspect of the present invention, provided is a method for screening an RNA aptamer against a target protein, comprising: (1) providing a screening cell, wherein the screening cell comprises a guide RNA, a target sequence, a selection marker, a fusion protein and a dCas protein, wherein the guide RNA comprises a recognition sequence and a nucleic acid aptamer random library having a predetermined length; the target sequence is complementary to the recognition sequence of the guide RNA; the selection marker is downstream of the target sequence and comprises a basal promoter and a selection marker gene; the fusion protein comprises the target protein and a transcriptional activation module, wherein a binding of the target protein to an RNA aptamer of the nucleic acid aptamer random library results in recruitment of the transcriptional activation module to the selection marker; and the dCas protein specifically recognizes the target sequence under the guidance of the guide RNA; (2) screening the screening cell using the selection marker gene; (3) collecting the screening cell that expresses the selection marker gene; and (4) lysing the screening cell, specifically amplifying a region containing the nucleic acid aptamer, and obtaining sequence information regarding the nucleic acid aptamer by sequencing. In some embodiments, step (4) further comprises an analysis and verification of the nucleic acid aptamer as obtained. In some embodiments, the dCas protein is a dCas9 protein or dUn1Cas12f1 protein. In some embodiments, the basal promoter is selected from mini-promoter-1 (SEQ ID NO: 28), mini-promoter-2 (SEQ ID NO: 27), mini-TK promoter (SEQ ID NO: 29), mini-CMV promoter (SEQ ID NO: 30) and Crystallin basal promoter (SEQ ID NO: 31). In some embodiments, the target protein is derived from a virus, a bacterial, a fungus or an animal. In some embodiments, the target protein is derived from a human. In some embodiments, the target protein is a green fluorescent protein or is derived from a RBD region of SARS-CoV-2 Spike protein. In some embodiments, the screening cell is a HEK293T cell or *E. coli.*

In some embodiments, the screening cell is obtained by transforming one or more plasmid vectors into a prokaryotic cell, wherein the one or more plasmid vectors comprise one or more selected from a group consisting of: the guide RNA; the target sequence and the selection marker; a nucleic acid sequence encoding the fusion protein; and a nucleic acid sequence encoding the dCas protein, wherein the guide RNA comprises a recognition sequence and a nucleic acid aptamer random library having a predetermined length; the target sequence is complementary to the recognition sequence of the guide RNA; the selection marker is downstream of the target sequence and comprises a basal promoter and a selection marker gene; the fusion protein comprises the target protein and a transcriptional activation module, wherein a binding of the target protein to an RNA aptamer of the nucleic acid aptamer random library results in the recruitment of the transcriptional activation module to the selection marker; and the dCas protein specifically recognizes the target sequence under the guidance of the guide RNA. In some embodiments, the screening cell is obtained by transforming a single plasmid vector into a prokaryotic cell, wherein the plasmid vector comprises the guide RNA; the target sequence; the selection marker; a nucleic acid sequence encoding the fusion protein; and a nucleic acid sequence encoding the dCas protein. In some embodiments, the prokaryotic cell is *E. coli.* In some embodiments, the method further comprises (5) subcloning the sequence of the nucleic acid aptamer obtained in step (4) to a plasmid vector and repeating steps (2) to (4).

In some embodiments, the screening cell is obtained through the following steps: (1.1) providing a cell that expresses the dCas protein, wherein the dCas protein specifically recognizes the target sequence under the guidance of the guide RNA, and wherein the cell further comprises the target sequence and the selection marker, the target sequence is complementary to the recognition sequence of the guide RNA, and the selection marker is downstream of the target sequence and comprises the basal promoter and the selection marker gene; (1.2) providing a viral expression vector comprising a nucleic acid encoding the guide RNA and the fusion protein, wherein the guide RNA comprises the recognition sequence and a nucleic acid aptamer random library having a predetermined length and the fusion protein comprises the target protein and the transcriptional activation module, wherein a binding of the target protein to an RNA aptamer of the nucleic acid aptamer random library results in recruitment of the transcriptional activation module to the selection marker; and (1.3) packaging the viral expression vector and infecting the cell with the packaged viral expression vector. In some embodiments, the method further comprises step (5): subcloning the sequence of the nucleic acid aptamer obtained in step (4) to the viral expression vector obtained in step (1.2) and repeating steps (1.3) and steps (2) to (4). In some embodiments, step (5) is repeated 1 to 3 times. In some embodiments, virus infection is performed at an MOI of 3 to 5 when step (1.3) is initially performed, and at an MOI of 0.1 to 0.3 when step (1.3) is re-performed. In some embodiments, in step (1.3) a virus titer of the packaged viral expression vector is determined using an antibiotic. In some embodiments, the viral expression vector is lentiviral expression vector. In some embodiments, the cell is a HEK293T cell.

Another aspect of the present invention provides a detection reagent or kit for detecting a SARS-CoV-2 virus, wherein the detection reagent or kit comprises an RNA aptamer specifically binding to S1 protein of the SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the SARS-CoV-2 virus is SARS-CoV-2 wild-type virus, or its Delta, Omicron BA.1 or BA.2 variants.

Another aspect of the present invention provides a method for detecting a SARS-CoV-2 virus, comprising contacting a sample to be detected with an RNA aptamer specifically binding to S1 protein of the SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the SARS-CoV-2 virus is SARS-CoV-2 wild-type virus, or its Delta, Omicron BA.1 or BA.2 variants.

Further provided is use of an RNA aptamer specifically binding to S1 protein of a SARS-CoV-2 virus in the preparation of a detection reagent or kit for detecting a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the SARS-CoV-2 virus is SARS-CoV-2 wild-type virus, or its Delta, Omicron BA.1 or BA.2 variants.

Another aspect of the present invention provides a method for neutralizing a SARS-CoV-2 virus in a subject, comprising administering to the subject in need thereof an effective amount of the pharmaceutical agent provided herein, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Further provided is use of a pharmaceutical agent in the preparation of a medicine for neutralizing a SARS-CoV-2 virus in a subject, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides a method for prevention or treatment of a SARS-CoV-2 virus infection, comprising administering to a subject in need thereof an effective amount of the pharmaceutical agent as provided herein, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides use of the pharmaceutical agent as provided herein in the preparation of a medicine for prevention or treatment of a SARS-CoV-2 virus infection in a subject, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides a method for neutralizing a SARS-CoV-2 virus in a subject, comprising administering to a subject in need thereof an effective amount of a pharmaceutical composition, wherein the pharmaceutical composition comprises a pharmaceutical agent and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides use of a pharmaceutical composition as provided herein in the preparation of a medicine for neutralizing a SARS-CoV-2 virus in a subject, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides a method for prevention or treatment of SARS-CoV-2 virus infection in a subject, comprising administering to the subject in need thereof an effective amount of a pharmaceutical composition as provided herein, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Another aspect of the present invention provides use of a pharmaceutical composition as provided herein in the preparation of a medicine for prevention or treatment of SARS-CoV-2 virus infection in a subject, wherein the pharmaceutical composition comprises a pharmaceutical agent as provided herein and a pharmaceutically acceptable excipient, wherein the pharmaceutical agent comprises an RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7. In some embodiments, the random region comprises a nucleic acid sequence set forth in SEQ ID NO: 2 or 7. In some embodiments, the constant sequence at 5' end of the RNA aptamer comprises a nucleic acid sequence set forth in SEQ ID NO: 8. In some embodiments, the constant sequence at 3' end of the RNA aptamer comprises a nucleic acid sequence set forth in SEQ ID NO: 9. In some embodiments, the constant sequence at 5' end of the RNA aptamer comprises a nucleic acid sequence set forth in SEQ ID NO: 8 and the constant sequence at 3' end of the RNA aptamer comprises a nucleic acid sequence set forth in SEQ ID NO: 9. In some embodiments, the RNA aptamer is chemically modified and the chemical modification is one or more modifications selected from a group consisting of fluorine substitution, O-methylation, PEGylation and cholesterol-PEGylation, wherein the PEGylation is selected from hexapolyethylene glycol to a PEG having a 40 kD molecule weight. In some embodiments, the pharmaceutical agent further comprises a viral or non-viral vector, wherein the vector comprises or carries the RNA aptamer specifically binding to S1 protein of the SARS-CoV-2 virus. In some embodiments, the viral vector is a lentiviral vector. In some embodiments, the non-viral vector is a plasmid. In some embodiments, the SARS-CoV-2 virus is SARS-CoV-2 wild-type virus, or its Delta, Omicron BA.1 or BA.2 variants.

Suitable routes of administration include parenteral administration (e.g., intramuscular, intravenous, or subcutaneous), oral, nasal spray, and nebulized inhalation. The pharmaceutical pharmaceutical agent or compositions of the present invention may be administered via multiple conventional methods, such as tracheal intubation, oral ingestion, inhalation, topical application, or injection through transdermal, subcutaneous, intraperitoneal, parenteral, intraarterial, or intravenous routes. Furthermore, the RNA aptamers, pharmaceutical agents, or compositions of the invention may be delivered via targeted drug delivery systems. In some embodiments, the RNA aptamers, pharmaceutical agents, or compositions are administered via intravenous injection.

Appropriate dosing is determined by clinicians based on parameters or factors known or suspected to influence treatment efficacy or anticipated therapeutic outcomes in the art. Typically, an initial dose is slightly below the optimal level, followed by incremental increases until the desired or maximal therapeutic effect is achieved relative to any adverse side effects. Critical diagnostic metrics include monitoring inflammatory symptoms or levels of inflammatory cytokines produced.

The pharmaceutical agents or compositions of the invention are administered via continuous delivery or at defined intervals (e.g., daily, weekly, or 1-7 times per week). Dosing may be provided via tracheal intubation, intravenous, subcutaneous, intraperitoneal, transdermal, topical, oral, nasal, rectal, intramuscular, intracerebral, or intraspinal routes. A preferred dosing regimen is one that maximizes therapeutic efficacy while avoiding significant undesirable side effects, either through dose magnitude or administration frequency.

### Experimental Cell Lines and Methods

Experimental Cell lines. HEK293T cell line used in this study was purchased from the American Type Culture Collection (ATCC). HEK293T cells were cultured in DMEM (Dulbecco's Modified Eagle Medium) supplemented with 100 U/ml penicillin/streptomycin and 10% fetal bovine serum (FBS). The 293T/dCas9 cell line is a monoclonal cell line generated by stable lentiviral integration of the dCas9 expression cassette into wild-type HEK293T cells. The CRISmers screening cell line dCas9/9×gLuc-puro is a monoclonal cell line derived by further stable lentiviral integration of the 9×gLuc-puro reporter cassette (9×gLuc-minPro-puro) into the 293T/dCas9 cell line. The HEK293T/hACE2 cell line is a polyclonal cell line established via stable lentiviral integration of the hACE2 expression cassette into wild-type HEK293T cells. All cell lines tested negative for mycoplasma contamination and were maintained in a humidified incubator at 37°C with 5% CO₂.

Construction of Expression Vectors. The construction methods for all expression vectors in this study followed protocols outlined in Molecular Cloning: A Laboratory Manual (Fourth Edition). The construction of expression vectors primarily involved the following steps: preparation of linearized vectors, generation of insert fragments, ligation of vector and insert fragments, plasmid transformation using *Escherichia coli* competent cells, colony PCR screening of *E. coli* bacterial clones, plasmid extraction, and sequencing and validation of the obtained plasmids. All lentiviral vector backbones used in this work were derived from Addgene.

High Purity Plasmid Extraction. To obtain large quantities of high-purity, high-quality plasmids, we employed the alkaline lysis method for plasmid extraction. Compared to rapid plasmid extraction protocols, high-purity plasmid purification enables stepwise removal of impurities such as endotoxins, RNA, proteins, and organic solvents. Plasmids purified via this high-purity method are suitable for applications including expression vector construction and diverse cellular experiments.

Culture of HEK293T cells. The HEK293T cell line is a commonly utilized and standardized cell line known for its ease of manipulation and widespread application in retroviral production, gene expression, and protein expression. The cultivation of this cell line primarily involved standard procedures well-established in the art for skilled practitioners including cell thawing, maintenance culture, passaging, and cryopreservation.

Non-liposome transient transfection of HEK293T cells. For transient transfection, cells should exhibit optimal viability and a defined confluency. Depending on experimental objectives, transfection may be performed in 96-well plates, 24-well plates, 6-well plates, or 10 cm dishes. In this study, luciferase reporter systems for validating aptamer affinity were predominantly conducted in 96-well plates, while lentiviral packaging was performed in 6-well plates or 10 cm dishes.

The required medium volume, transfection reagent, and DNA amount vary according to the surface area of culture wells, as detailed in the table below (summarizing parameters relevant and commonly used in the current study; minor subjective variations may exist in the data).

**Table 1. Reagents used for non-liposome transient transfection of HEK293 T cells**

| Culture Vessels | Cell seeding density | Serum-free medium for dilution | Transfection reagent/DNA amount |
|---|---|---|---|
| 96-well plate | 2.5×10⁴ | 10 µl | 0.6 µl /0.2 µg |
| 24-well plate | 15.625×10⁴ | *50* µl | 2.4 µl /0.8 µg |
| 6-well plate | 100×10⁴ | 250 µl | 9 µl /3 µg |
| 10 cm dish | 800×10⁴ | 500 µl | 51 µl /17 µg |

Transfection protocols may be selected as adherent or suspension transfection based on the purpose of the experiment. Adherent transfection is suitable for common cell lines such as HEK293T, while suspension transfection enhances contact between transfection complexes and cells to improve transfection efficiency, making it ideal for challenging transfections or experiments demanding high efficiency. Below is an exemplary workflow for adherent transfection in 96-well plates.
(1) Cell seeding one day prior to transfection: The designated area of the 96-well plate was pre-coated with 0.1% gelatin. HEK293T cells or target cells (with robust growth kinetics) were seeded at a density of 2.5×10⁴ cells/well into the coated plate. Cell confluency was maintained above 60% (optimal for assays at 48 h post-transfection; avoid over-confluence) on the day of transfection.
(2) On the transfection day: 200 ng DNA was mixed with 10 µl serum-free medium (adjust ratios for multiple DNA constructs while keeping total DNA constant). PEI transfection reagent was diluted in 10 µl serum-free medium, mixed thoroughly, and incubated at room temperature (RT) for 5 min. The diluted PEI was added dropwise to the DNA solution, mixed gently, and incubated at RT for 20 min. Add 20 µl of the PEI-DNA complex to each well containing cells and medium. The mixture was distribute evenly by crosswise agitation, then cultured at 37°C with 5% CO₂. After 6 h, the medium was replaced with fresh complete medium followed by continued incubation. For transfections in 24-well plates, 6-well plates, or 10 cm dishes, half of the medium was replaced with fresh 10% FBS/DMEM before adding the transfection complex.
(3) Post-transfection (48 h): Relevant assays or downstream treatments were performed at this timepoint.

Establishment of stable cell lines mediated by lentiviruses. The present study established three stable cell lines: 293T/dCas9 (monoclonal), dCas9/9×gLuc-puro (monoclonal), and HEK293T/hACE2 (polyclonal). The workflow involved HEK293T cell seeding, lentiviral packaging, viral harvest, target cell preparation, lentiviral infection, cell line screening, and validation. Monoclonal lines required single-cell colony isolation post-screening, whereas polyclonal lines did not.
(1) HEK293 T Cell Seeding: One day prior to transfection, HEK293 T cells (with robust growth kinetics) were seeded at a density of 1×10⁶ per well into 0.1% gelatin-precoated 6-well plates. Cell confluency was maintained >80% on the day of transfection.
(2) Lentiviral Packaging: The non-liposomal transient transfection protocol was followed using three plasmids: pRRL lentiviral vector (containing the target gene expression cassette), pCMV-VSV-G (expressing envelope protein VSVG), and psPAX2 (expressing packaging proteins GAG/POL) at a mass ratio of 10:9:1 (pRRL:pCMV-VSV-G:psPAX2). DNA was diluted in 50 µl serum-free medium; PEI transfection reagent was diluted in 50 µl serum-free medium, mixed thoroughly, and incubated at RT for 5 min. The diluted PEI was added dropwise to the DNA solution, mixed gently, and incubated at RT for 20 min. Medium in the 6-well plate was replaced with 1 ml fresh 10% FBS/DMEM before adding the 100 µl transfection complex, followed by crosswise agitation and culture at 37°C, 5% CO₂. The medium was replaced after 6-8 h.
(3) Lentivirus Collection: At 48 hours post-transfection, the cell culture supernatant was collected. Subsequently, fresh culture medium was slowly added along the inner wall of the culture vessel and incubation continued for an additional 24 hours. The harvested viral supernatant was filtered through a 0.45 µm filter, and the filtrate was temporarily stored at 4°C. At 72 hours post-transfection, the cell culture supernatant was collected again, filtered it through a 0.45 µm filter, and combined with the previously collected 48-hour viral supernatant. The pooled viral solution could be aliquoted and stored at -80°C. For short-term use, it may be temporarily stored at 4°C. Virus-contaminated pipette tips and other experimental equipment must undergo autoclaving (high-temperature and high-pressure sterilization) before disposal in designated biohazard waste collection bags for centralized processing.
(4) Preparation of Target Cells for Infection: Viral infection was similar to transient cell transfection and could be performed via adherent or suspension infection, following the same principles. Using adherent infection as an example: One day prior to infection, target cells (with rapid growth and optimal viability) were seeded into a 24-well plate pre-coated with 0.1% gelatin at a density of 1×10⁵ cells per well. One well was reserve as an uninfected blank control group.
(5) Lentiviral Infection: The medium was aspirated from the 24-well plate along the wall and viral supernatant containing 8 µg/ml Polybrene (an infection enhancer) was added. The culture volume was adjusted to 250 µl, gently mixed by swirling, and incubated. After 8-12 hours, the viral supernatant was aspirated along the wall and fresh medium was gently replenished along the wall, followed by continued incubation. The blank control group underwent identical procedures but was not exposed to lentivirus. Following lentiviral infection, the target gene integrated into the host cell genome. The target gene typically included an antibiotic resistance marker for subsequent cell selection.
(6) Monoclonal Cell Line Screening: At 48 hours post-viral infection, when cell density was high, cells were passaged to a lower density and the original medium was replaced with fresh medium containing the appropriate antibiotic concentration. The antibiotic-containing medium was refreshed every two days. Different antibiotics exhibit distinct mechanisms of action, and cellular sensitivity to antibiotics varies; thus, pre-experimental optimization of antibiotic concentration and exposure time was required. The blank control group, lacking lentivirus-mediated antibiotic resistance gene expression, underwent complete cell death over the screening period. Survival in the infected group was monitored and the complete death of blank control cells was used as the endpoint.
(7) Monoclonal Cell Colony Picking: Typically, after 10-12 days of antibiotic selection, successfully infected cells formed compact, clonal colonies. Under a stereomicroscope, a 200 µl pipette tip was used to gently scrape or aspirate individual colonies and transfer them to a 24-well plate for expansion. Isolated, smooth-edged, non-overlapping colonies with robust growth was prioritized. The number of colonies picked depends on lentiviral infection efficiency, with 20-40 colonies recommended. Establishment of polyclonal cell lines did not require single-colony isolation.
(8) Validation of Stable Cell Lines: When the monoclonal cell clusters transferred to the 24-well plate reached sufficient density, a portion was cryopreserved, while the remainder was subjected to validation. The validation methodology depends on the characteristics of the delivered transgene, such as the inserted sequence, fluorescent markers, protein tags, or functional properties. Corresponding analytical techniques included genotypic characterization/Sanger sequencing, flow cytometry, Western blot, and microplate reader assays.
(9) CRISmers Screening Cell Line Establishment and Validation: The dCas9/9×gLuc-puro cell line was a monoclonal cell line generated by further stable integration of the 9×gLuc-puro reporter cassette into the 293T/dCas9 cell line via lentiviral transduction. For validation of dCas9/9×gLuc-puro cell lines, individual monoclonal colonies were independently infected with two lentiviral constructs: lenti-hU6-gLuc sgRNA-1.2 GFP apt-EF1a-NLS-GFP-V-PH (Positive Control, PC); and lenti-hU6-gLuc sgRNA-1.2 blank-EF1a-NLS-GFP-V-PH (Negative Control, NC). At 48 hours post-infection, each monoclonal cell line was treated with varying puromycin concentrations (0.5 µg/ml, 1 µg/ml, 2 µg/ml) to select for resistant clones. The number of surviving clones post-selection was quantified for each monoclonal line. A higher PC/NC ratio indicates superior cell line activity and sensitivity. Additionally, the number of surviving clones in the NC group was monitored; fewer surviving clones correlate with lower background interference.

Luciferase Reporter Experiment. Luciferase reporter experiment was used in this study to assess the CRISmers system and to screen the affinities of nucleic acid aptamers subsequently identified.
(1) Transfection of HEK293T cells
   (a) Validation of CRISmers system: The steps were consistent with the non-liposomal transient transfection protocol for 96-well plates described previously. Using PEI transfection reagent, the following plasmids (based on the 293T/dCas9 stable cell line) were transiently transfected into designated wells of a 96-well plate: p1xgLuc target firefly luciferase or p9xgLuc target firefly luciferase, phU6-gLuc sgRNA-1.1 GFP aptamer or phU6-gLuc sgRNA-1.2 GFP aptamer, pGFP-VPH and pCMV-renilla luciferase. pCMV-renilla luciferase was added 20 ng, with the remaining three plasmids mixed at a 1:1:1 molar ratio. At 6-8 hours post-transfection, the medium was replaced with fresh 10% FBS/DMEM medium.
   (b) Assessment of Nucleic Acid Aptamer Affinity: The procedure aligns with the non-liposomal transient transfection protocol for 96-well plates described previously. Using PEI transfection reagent, the following plasmids (based on the 293T cell line) were transiently transfected into designated wells of a 96-well plate: phPGK-dCas9, p9xgLuc target gaussia luciferase, and phU6-gLuc sgRNA-1.2 X aptamer-EF1a-NLS-RBD of S1-VPH, mixed at a 1:1:1 molar ratio. Prior to affinity assessment, the blank expression vector (phU6-gLuc sgRNA-1.2 BsmBI-BsmBI-polyT-EF1a-NLS-linker-EcoRI-RBD-EcoRI-linker-VP64-P65-HSF1-NLS-linker-P2A-linker-Zeo-WPRE) was constructed. The nucleic acid aptamer sequences were inserted into the gLuc sgRNA stem-loop 2 (1.2) backbone region using BsmBI restriction enzyme digestion. Due to the large number of aptamers requiring parallel comparison, the constructed expression vectors can be directly used for transfection following rapid plasmid miniprep; large-scale high-purity plasmid preparation is unnecessary. At 6-8 hours post-transfection, the medium was replaced with fresh 10% FBS/DMEM medium.
(2) Luciferase Activity Assay: Luciferase activity was detected using dual-luciferase reporter kit available from Vigorous at 48 hours post-transfection.
   (a) CRISmers System Validation: Cell Lysis: At 48 hours post-transfection, the medium was carefully aspirated from the 96-well plate. Using a multichannel pipette, 30 µl of 1x universal lysis buffer was added to each well. The plate was incubated on a microplate shaker or low-speed orbital shaker at room temperature for 5-10 minutes. After complete lysis, 20 µl of lysate was transferred to a new 96-well plate using a multichannel pipette. Firefly Luciferase Activity Assay: using a multichannel pipette, 20 µl of Firefly Luciferase Assay Reagent (Fassay Reagent) was dispensed into the bottom of each well. The plate was gently tapped 3-5 times to mix, then the chemiluminescence was immediately measured using a microplate reader. The luminescence value was recorded as the firefly luciferase activity signal. Renilla Luciferase Activity Assay: Using a multichannel pipette, 20 µl of Renilla Luciferase Assay Reagent (Rassay Reagent) was dispensed into the bottom of each well. The plate was gently tapped 3-5 times to mix, then the chemiluminescence was immediately measured using a microplate reader. The luminescence value was recorded as the Renilla luciferase activity signal.
   (b) Assessment of Nucleic Acid Aptamer Affinity: At 48 hours post-transfection, 20 µl of cell culture supernatant was aspirated from the 96-well plate using a multichannel pipette and transferred to a new black-well 96-well plate. Subsequently, 20 µl of Rassay Reagent was added to the bottom of each corresponding well using a multichannel pipette. The plate was gently tapped 3-5 times along the walls to ensure mixing and immediately subjected to chemiluminescence measurement using a microplate reader. The recorded luminescence value represented the Gaussia luciferase activity signal. Due to the high number of nucleic acid aptamers requiring validation during secondary screening, Gaussia luciferase (a secreted luciferase detectable in the cell supernatant) was employed to streamline the workflow and enhance detection efficiency.
(3) Luciferase Activity Assay
   (a) CRISmers System Validation: The data were calculated as the ratio of Firefly luciferase activity to Renilla luciferase activity, where Renilla luciferase was utilized as internal experimental control. Subsequently, all experimental data were standardized by dividing them by the average value of the control group.
   (b) Assessment of Nucleic Acid Aptamer Affinity: All experimental data were standardized by dividing them by the average value of the control group.

Construction of a Randomized Oligonucleotide Library for Nucleic Acid Aptamers

In this study, to ensure experimental feasibility and operational convenience, a blank expression vector (designated as phU6-gLuc sgRNA-1.2 BsmBI-BsmBI-polyT-EF1a-NLS-linker-EcoRI-EcoRI-linker-VP64-P65-HSF1-NLS-linker-P2A-linker-Zeo-WPRE) was constructed. First, the open reading frame (ORF) of the target protein of interest for screening was inserted into the lentiviral blank expression vector using the restriction enzyme EcoRI. Subsequently, a synthesized 20-bp random oligonucleotide library was inserted into the expression vector via restriction enzyme BsmBI, generating a 20-bp oligonucleotide screening library specific to the target protein. The library construction workflow was divided into the following key steps: PCR amplification of the randomly synthesized oligonucleotide library, enzymatic digestion of the lentiviral vector, Gibson assembly, electroporation of the assembled library into competent cells, lentiviral packaging of the library and titer determination of the packaged lentiviral particles.
(1) PCR Amplification of the Randomized Oligonucleotide Library: The synthesized oligonucleotide library was composed of 100-bp sequences, with a 20-bp randomized region (A, T, C, G) flanked by 40-bp homology arms at the 5' and 3' ends (AGCAAGTTAAAATAAGGCTAGTCCGTTATCAACTTGGCCAnnnnnnnnnnnnnnnnnnnnC TGCAGGGCCAAGTGGCACCGAGTCGGTGCTTTTTATCGA; SEQ ID NO: 10) for vector homology-directed insertion. The lyophilized oligonucleotide library powder was centrifuged at 12,000 rpm for 5 minutes to pellet particles adhering to the tube walls, minimizing sample loss and ensuring accurate concentration measurement. The pellet was dissolved in sterile nuclease-free water to a final concentration of 1 µg/µl, followed by dilution to 0.1 ng/µl, and stored on ice for immediate use. To ensure amplification fidelity and library coverage, the PCR reaction system and cycling conditions for the randomized oligonucleotide library were optimized and differed from standard PCR protocols, as detailed in the table below.

**Table 2. PCR amplification system for the oligo library**

| Reagents | Volume |
|---|---|
| NEB Hot Smart PCR Master Mix (2×) | 25 µl |
| Oligo pool | 1 µl |
| 10 µM oligo-Primer F | 2.5 µl |
| 10 µM oligo-Primer R | 2.5 µl |
| dH₂O | 19 µl |

**Table 3. PCR amplification procedure for the oligo library**

| Procedure | Time | |
|---|---|---|
| Initial denaturation | 98°C, 30 s | |
| Denaturation | 98°C, 30 s | |
| Annealing | 63°C, 30 s | 15 cycles |
| Extension | 72°C, 30 s | |
| Final extension | 72°C, 5 min | |
| | 4°C, ∞ | |

The primer sequences used for PCR amplification of the oligo library (5' to 3') were designed as follows: oligo-Primer F: TATGTTTAAGAGCTAGAAATAGCAAGTTAAAATAAGGCTA (SEQ ID NO: 11), oligo-Primer R: CCATCTTTGCAAAGCTTATATCGATAAAAAGCACCGACTC (SEQ ID NO: 12). After amplification was completed, 2 µl of the product was analyzed by agarose gel electrophoresis to verify the expected product size (140 bp) and specificity (absence of nonspecific bands). A 2% agarose gel was utilized to resolve small DNA fragments effectively. The amplified product was purified using the QIAquick PCR Purification Kit according to the manufacturer's instructions. If the purified sample concentration was deemed insufficient, additional parallel amplification reactions were performed to ensure adequate library material for downstream ligation. Purified samples were stored at 4°C for short-term use. For long-term preservation, samples were stored at -20°C or -80°C to prevent degradation.

(2) Enzymatic Digestion of the Lentiviral Vector: The lentiviral expression vector containing the SARS-CoV-2 S protein RBD open reading frame (ORF) was digested and dephosphorylated using the BsmBI restriction enzyme. Following digestion, 2 µl of the reaction product was analyzed by 1% agarose gel electrophoresis to validate digestion efficiency, with 1 µl of undigested original vector included as a control. Compared to the supercoiled original vector, the digested vector appeared linearized, migrating slightly higher on the gel. The linearized vector was purified using the QIAquick Gel Extraction Kit according to the manufacturer's instructions. To remove residual salts and impurities introduced during enzymatic digestion and to enhance Gibson assembly efficiency, the purified vector was concentrated via isopropanol precipitation. The reaction setup for precipitation was configured as detailed in the table below.

**Table 4. Reaction Setup for Isopropanol Precipitation**

| Reagents | Volume |
|---|---|
| Purified product of gel cutting | 20 µl |
| isopropanol | 20 µl |
| GlycoBlue Coprecipitant | 0.2 µl |
| 5 M NaCl | 0.4 µl |

The reaction mixture was vortexed thoroughly and incubated at room temperature for 15 minutes. It was then centrifuged at 12,000 rpm for 15 minutes, resulting in a blue pellet at the bottom of the tube. The supernatant was carefully discarded, and the pellet was washed twice with pre-chilled 80% (vol/vol) ethanol (-20°C). After each wash, the sample was centrifuged briefly, residual ethanol was removed, and the pellet was air-dried for 5 minutes. Depending on the pellet size, a variable volume of dH2O was added to dissolve the DNA, followed by incubation in a 55°C metal heating block to ensure complete resuspension. If the purified DNA concentration was insufficient, multiple isopropanol precipitation reactions were pooled and processed proportionally. The purified DNA was stored at 4°C for short-term use. For long-term preservation, samples were stored at -20°C or -80°C to prevent degradation.

(3) Gibson Assembly. The Gibson reaction system was established as follows and incubated in a PCR apparatus for 1 h at 50°C.

**Table 5. Reaction system for Gibson Assembly**

| Reagents | Mass/Volume |
|---|---|
| Gibson Assembly Master Mix | 15 µl |
| PCR-amplified Random library | 60 ng |
| Linearized vector | 200 ng |
| dH₂O | To 20 µl |

Multiple parallel Gibson assembly reactions were performed to ensure sufficient library material for subsequent electroporation. A blank control group was included, where the volume of the PCR-amplified randomized library was replaced with an equal volume of deionized water, to assess background effects and calculate library coverage. To remove residual salts and contaminants introduced during Gibson assembly and enhance electroporation efficiency, the ligated library was concentrated via isopropanol precipitation. The reaction mixture was vortexed thoroughly and incubated at room temperature for 15 minutes, followed by centrifugation at 12,000 rpm for 15 minutes, yielding a blue pellet at the tube bottom. The supernatant was discarded, and the pellet was washed twice with pre-chilled 80% (vol/vol) ethanol (-20°C). After brief centrifugation, residual ethanol was removed, and the pellet was air-dried for 5 minutes. Depending on the pellet size, a variable volume of dH2O was added to dissolve the DNA, followed by incubation in a 55°C metal heating block. If the purified library concentration was insufficient, multiple isopropanol precipitation reactions were pooled and processed proportionally. The purified library was stored at 4°C for short-term use. For long-term preservation, samples were stored at -20°C or -80°C to prevent degradation. The resuspended ligation product was adjusted to a final concentration of 100-150 ng/µl to optimize electroporation efficiency.

### (4) Library Electroporation

(a) Electroporation cuvette pre-cooling: The electroporation cuvette (0.1 cm) was inserted into crushed ice, the ice surface was compacted, and it was allowed to stand for later use.
(b) SOC medium preheating: The antibiotic-free SOC medium was placed in a 37°C oven for preheating and allowed to stand for later use.
(c) Electroporation: The electroporation parameters of the electroporator were set according to the manufacturer's instructions. Electrocompetent cells were retrieved from the - 80°C freezer and placed on compacted ice. When the cells had just thawed, 2 µl of the aforementioned ligation product was added, gently pipetted 2-3 times to mix, and the mixture was rapidly transferred to the pre-cooled electroporation cuvette, followed by closing the cuvette lid. Moisture on the exterior of the electroporation cuvette was wiped off with a towel, and the cuvette was promptly placed into the electroporation chamber for electroporation. Immediately after electroporation, 1 ml of preheated SOC medium was added to the electroporation cuvette, followed by incubation at 37°C with shaking at 220 rpm for 1 hour. During the electroporation process, bubbles were avoided, and moisture on the cuvette walls was thoroughly dried to ensure that the electroporation procedure was performed swiftly and seamlessly.
(d) Plating: The cultured bacterial suspension was evenly spread onto agar plates at a volume of 250 µl per plate, followed by overnight incubation at 37°C.
(e) Colony counting: The bacterial colonies were counted to evaluate transformation efficiency and library coverage. Prior to electroporation of the final library, preliminary experiments were conducted to estimate background effects and transformation efficiency. Due to the excessively high initial sequence count (4²⁰) in the first round, existing library construction technologies were unable to cover all sequences, and efforts were focused on maximizing the number of clones obtained. Starting from the second round of library construction, full sequence coverage was achieved using current library construction technologies, with a minimum of 500-fold coverage ensured.
(f) Bacterial harvesting: After assessing the number of bacterial clones obtained from electroporation, cells were harvested in a laminar flow hood using a microspatula or cell scraper. The harvested cells were incubated at 37°C with shaking at 220 rpm for 2-3 hours.
(g) High-purity plasmid extraction: Following bacterial amplification, cells were collected and subjected to high-purity plasmid extraction. The extracted plasmids were stored at -80°C for long-term preservation.

Packaging of Lentiviral Libraries for Nucleic Acid Aptamer Oligonucleotide Randomized Sequences and Lentiviral Titer Determination:

### (1) Lentiviral Library Packaging

The lentiviral library packaging procedure was largely consistent with the lentiviral packaging process described earlier for establishing stable cell lines via lentiviral transduction. Due to the high demand for lentiviral particles, the packaging process was performed simultaneously across multiple 10 cm dishes. The protocol aligned with non-liposomal transient transfection and involved three plasmids: the pRRL lentiviral vector plasmid containing the randomized sequence library, the pCMV-VSV-G plasmid expressing the VSV-G envelope protein, and the psPAX2 plasmid encoding the lentiviral packaging proteins GAG and POL, with a mass ratio of 10:9:1.
(a) Day 1: The 10 cm dishes were pre-coated with 0.1% gelatin. Rapidly proliferating and morphologically healthy HEK293T cells were seeded into 10 cm dishes at a density of 8 x 10⁶ cells per dish. Cell confluency was maintained above 80% on the day of transfection to ensure optimal efficiency.
(b) Day 2: DNA was diluted in 500 µl of serum-free medium and mixed thoroughly. Separately, the transfection reagent PEI was diluted in 500 µl of serum-free medium, mixed uniformly, and incubated at room temperature for 5 min. The diluted PEI was then added dropwise to the diluted DNA solution, mixed gently, and incubated at room temperature for 20 min. During the incubation interval, the culture medium in the 10 cm dishes containing cells to be transfected was replaced with 5 ml of fresh 10% FBS/DMEM medium. After incubation, 1000 µl of the DNA-PEI transfection complex was added to the wells containing cells and medium. The mixture was swirled in a crosshatch pattern to ensure even distribution, and the dishes were placed in a 37°C, 5% CO₂ cell culture incubator. Fresh medium was replaced after 6-8 hours, and the dishes were returned to the incubator for continued culture.
(c) Lentiviral Harvesting: At 48 hours post-transfection, the cell culture supernatant was collected. Fresh medium was then slowly added along the walls of the dishes, and the cells were cultured for an additional 24 hours. The harvested viral suspension was filtered through a 0.45 µm filter, and the filtrate was temporarily stored at 4°C. At 72 hours post-transfection, the supernatant was collected again, filtered, and combined with the viral suspension collected at 48 hours. The pooled viral stock was aliquoted and stored at -80°C for long-term preservation. For short-term use, aliquots could be stored at 4°C. Pipette tips and instruments exposed to the viral suspension were autoclaved and disposed of in dedicated biohazard waste collection bags.

### (2) Viral Titer Determination

Viral titer was determined using the Zeocin resistance gene encoded in the library vector. Healthy dCas9/9×gLuc-puro cells were seeded into a 12-well plate at a density of 3×10⁶ cells per well (defined as the "initial cell count"). Subsequently, 0 µl, 25 µl, 50 µl, 100 µl, 200 µl, and 400 µl of the lentiviral library stock were added to the cells for suspension centrifugation-mediated infection, with 8 µg/ml polybrene included to enhance infection efficiency. The plate was centrifuged at 37°C and 2400 rpm for 2 hours, followed by incubation in a 37°C cell culture incubator for 12-14 hours. At 48 hours post-infection, infected cells were re-seeded into new 12-well plates at a density of 1×10⁴ cells per well and cultured in medium containing 500 µg/ml Zeocin. Control groups included virus-infected cells without antibiotic and uninfected cells with antibiotic. After 5-6 days of continuous antibiotic selection, uninfected cells with antibiotic were completely eliminated, while virus-infected cells without antibiotic reached 80-90% confluency. Cells were then trypsinized and counted. The cell counts from antibiotic-treated, virus-infected groups were defined as "viable cell counts", and those from antibiotic-free, virus-infected groups as "control cell counts". Lentiviral titer was calculated using the formula: Titer (IFU/ml) = (Viable cell counts × Initial cell counts) / (Control cell counts x Virus volume in ml). The final titer was reported as the average of valid measurements. Notably, experimental groups showing >50% cell survival were excluded from titer averaging to avoid skewed results.

### Lentiviral Screening of Nucleic Acid Aptamer Oligonucleotide Randomized Sequence Libraries

Lentiviral library screening was performed using the monoclonal cell line #4-53 dCas9/9×gLuc-puro. The screening procedure was largely consistent with the lentiviral screening process described for establishing stable cell lines via lentiviral transduction. Due to the high cellular demand for library screening, the process was conducted across multiple 10 cm dishes. During the initial round of library infection and screening, a moderate MOI (3-5) was utilized to ensure broad coverage of functional sequences while minimizing screening workload. In subsequent rounds of sub-library screening, a low MOI (0.1-0.3) was employed to ensure that most cells received only one aptamer sequence. Following 48 hours of viral infection, the cells were passaged and maintained at 30%-40% confluency. The medium was replaced with 2 µg/ml puromycin-containing medium for selection, which was refreshed every 2-3 days. After approximately 10 days of continuous selection, clonal clusters derived from single cells became visible. All clones were manually picked using a stereomicroscope and lysed with cell genomic PCR lysis buffer. Lysis was performed by incubation at 50°C for 1 hour followed by 95°C for 15 minutes. A 1 µl aliquot of the lysate was directly used for genomic PCR amplification. The genomic PCR amplification system, thermal cycling parameters (annealing, extension, and final extension for 20 cycles), primers, post-amplification agarose gel electrophoresis validation, and purification steps were identical to those described for the PCR amplification of the oligonucleotide randomized library. Purified PCR products were submitted to Genewiz for deep sequencing.

### Deep Sequencing Data Analysis

Upon obtaining the deep sequencing results for each round, the sequencing quality was first evaluated to verify compliance with expected standards, primarily focusing on total reads output and base call accuracy (Q30).

For data analysis, aptamer sequences with mismatched homologous regions at both termini were filtered out. Subsequently, adapter sequences and homologous regions flanking the aptamers were removed, yielding candidate aptamer sequences. To identify high-affinity sequences, aptamers for secondary luciferase reporter assay screening were selected based on two criteria. Frequency ranking: independent sequences were sorted by their occurrence frequency in descending order. The top 0.1% of high-frequency sequences from the second screening round were selected for validation. Enrichment index: The enrichment index of each unique sequence between two screening rounds was calculated (defined as the ratio of its occurrence count in Round 2 to Round 1). Sequences were ranked by this index, and the top 15 with the highest enrichment indices were chosen for validation.

### SARS-CoV-2 Pseudovirus Packaging Experiment

The packaging of SARS-CoV-2 pseudovirus was generally consistent with the aforementioned lentiviral packaging procedure. The pseudovirus packaging system involved five plasmids: pCMV-firefly luciferase, pSpike or pDelta or pOmicron, pTAT, pRev, and pGAGpol.
(1) One day prior to pseudovirus packaging: A 6-well plate was pre-coated with 0.1% gelatin to enhance cell adhesion. HEK293T cells exhibiting rapid growth and optimal condition were selected and seeded into the gelatin-coated 6-well plate at a density of 1×10⁶ cells per well. The plate was incubated overnight in a cell culture incubator maintained at 37°C with 5% CO₂.
(2) Pseudovirus packaging: Prior to transfection, the original medium in the 6-well plate was replaced with 1 ml of fresh 10% FBS/DMEM medium. The pseudovirus packaging process was executed through non-liposomal transient transfection of the following five plasmids: 2 µg pCMV-firefly luciferase, 0.4 µg pSpike or 0.4 µg pDelta or 0.4 µg pOmicron, 0.2 µg pTAT, 0.2 µg pRev, and 0.2 µg pGAGpol. The plasmid mixture was diluted in 200 µl serum-free DMEM. Separately, 9 µl PEI transfection reagent was diluted in 200 µl serum-free DMEM, mixed gently, and incubated at room temperature for 5 minutes. After incubation, the diluted PEI solution was added dropwise to the diluted plasmid mixture, mixed thoroughly, and incubated at room temperature for 20 minutes. Following incubation, 400 µl of the plasmid-PEI complex was added dropwise along the wall of each well. The plate was gently swirled in a cross pattern to ensure uniform distribution and then returned to the 37°C, 5% CO₂ incubator for 6-8 hours. The medium was then replaced with fresh 10% FBS/DMEM.
(3) Pseudovirus Collection: After 48 hours of cell culture, the cell supernatant was carefully aspirated using a syringe and filtered through a 0.45 µm filter column. Pseudoviruses collected at the same time point were pooled for storage. The 48-hour pseudovirus was temporarily stored at 4°C. Following the 48-hour collection, 2 ml of fresh 10% FBS/DMEM medium was gently added along the wall of the well, and the culture was continued for an additional 24 hours (totaling 72 hours). At the 72-hour time point, the supernatant was similarly aspirated with a syringe and filtered through a 0.45 µm filter column. Pseudoviruses collected at both 48-hour and 72-hour time points were combined for storage. The pooled pseudovirus was aliquoted and cryopreserved at -80°C. Pipette tips and experimental tools that came into contact with the pseudovirus during collection were segregated, collected separately, and autoclaved for disposal.

### Protein-based ELONA Assay

In this study, the affinity and detection potential of selected aptamers were evaluated using the Enzyme-Linked Oligonucleotide Assay (ELONA). Both protein-based ELONA and pseudovirus-based ELONA experiments were conducted.
(1) Plate Coating: One day prior to detection, proteins at varying concentrations (dissolved in sterile water) were added to a high-binding 96-well microplate at a total volume of 100 µl per well. The plate was sealed with a plastic film and incubated overnight at 4°C. (For dissociation constant (Kd) determination, the RBD protein coating concentration was 250 ng/well. For protein sensitivity testing, RBD coating concentrations were 0.001 µg, 0.01 µg, 0.1 µg, 0.2 µg, and 0.4 µg, respectively.)
(2) Blocking: The incubation solution was aspirated using a vacuum aspirator. The coated wells were washed three times with washing buffer using a multichannel pipette, ensuring residual liquid was fully removed without touching the well bottom. After washing, blocking solution was added and incubated at 37°C for 30 min. After blocking, the wells were washed three additional times under identical conditions. (Washing buffer: PBS + 0.05% (vol/vol) Tween-20, pH 7.4; Blocking solution: Washing buffer + 2% (weight/vol) BSA).
(3) Aptamer Incubation: Biotin-modified aptamers at varying concentrations, diluted in sterile nuclease-free water, were added to the wells and incubated at different temperatures (4°C, 23°C, or 37°C) for 1 h. After incubation, the solution was carefully aspirated along the wall using a vacuum aspirator. The wells were washed three times with washing buffer using a multichannel pipette, ensuring residual liquid was fully removed without contacting the well bottom. Pipette tips were replaced between each step.
(4) Streptavidin-HRP Incubation: A 0.1 µg/ml Streptavidin-HRP solution, prepared in sterile water, was added to the wells and incubated at room temperature for 30 min. Subsequently, the solution was aspirated along the wall using a vacuum aspirator. The wells were washed three times with washing buffer using a multichannel pipette, ensuring complete removal of residual liquid while avoiding contact with the well bottom. Pipette tips were replaced during each aspiration and washing step.
(5) Substrate Incubation: 200 µl of substrate solution was added to each well and incubated at room temperature for 20 min. During this period, wells with strong signals developed a blue coloration.
(6) Reaction Termination: 50 µl of stop solution was added to each well, followed by incubation at room temperature for 2-5 min. The color of wells with strong signals shifted from blue to yellow upon termination.
(7) Signal Detection: The OD450 absorbance was measured using a microplate reader. Higher OD450 readings indicated stronger affinity binding between the aptamers and the target protein.
(8) Data Analysis: The dissociation constant (Kd) in this study was calculated using the equation Y = BmaxX / (Kd + X), where Y represented the average OD450 value, Bmax denoted the maximum OD450 value, and X corresponded to the concentration of the biotin-modified aptamer. The Kd values were automatically analyzed by GraphPad Prism software.

### Pseudovirus-Based ELONA Assay

Virus Coating: One day prior to detection, pseudovirus particles with varying titers (TCID50) were added to a high-binding 96-well microplate at a total volume of 100 µl per well. The plate was sealed with plastic film and incubated overnight at 4°C. Subsequent steps were identical to the aforementioned "protein-based ELONA assay." Notably, pipette tips and experimental tools exposed to pseudoviruses were autoclaved and disposed of in dedicated biohazard waste collection bags. Pseudovirus titer quantification was performed according to the quantitative detection method published by Youchun Wang's team.

### Gel Shift (Electrophoretic Mobility Shift Assay, EMSA)

The experiment was performed according to the Chemiluminescent EMSA Kit (Beyotime Biotechnology) manufacturer's instructions. Briefly, fixed amounts of biotinylated aptamer (1 pmol) were incubated with varying ratios of SARS-CoV-2 wild-type or variant Omicron BA.2 RBD protein (1:0, 1:1, 1:10, 1:20, 1:50) in EMSA binding buffer at room temperature for 30 minutes. In parallel, fixed amounts of biotinylated aptamer (1 pmol) were incubated with SARS-CoV-2 RBD protein at a 1:25 ratio, along with varying ratios of cold probe (non-biotinylated aptamer: 1:1, 1:2, 1:100, 1:200), in EMSA binding buffer at room temperature for 30 minutes. Similarly, fixed amounts of biotinylated aptamer (1 pmol) were incubated with SARS-CoV-2 variant Omicron BA.2 RBD protein at a 1:50 ratio, combined with varying ratios of cold probe (non-biotinylated aptamer: 1:1, 1:2, 1:100, 1:200), in EMSA binding buffer at room temperature for 30 minutes. Following incubation, the reaction mixtures were combined with loading buffer and electrophoresed on a 4% polyacrylamide gel at 100 V for 1 hour. The samples were then transferred to a positively charged nylon membrane. After electrophoretic transfer, cross-linking was performed using a handheld UV lamp for 5-10 minutes. Biotinylated aptamer signals were detected via a chemiluminescent detection system.

### SARS-CoV-2 Live Virus Neutralization Assay

To evaluate the inhibitory efficacy of selected RNA aptamers against SARS-CoV-2, and in alignment with current epidemiological trends, RNA aptamers #2-1-18 and #5-2-15 were tested for their neutralization activity against the SARS-CoV-2 Delta variant, Omicron BA. 1 variant, and Omicron BA.2 variant. All procedures involving live viruses were conducted in a Biosafety Level-3 (BSL-3) laboratory at the Shenzhen Center for Disease Control and Prevention, strictly adhering to BSL-3 protocols. The following viral strains were utilized: SARS-CoV-2 Delta variant: SARS-CoV-2 B.1.617.2 strain isolated by the Shenzhen Center for Disease Control and Prevention (designated as SZ/09/2022/SARS-CoV-2(D)). SARS-CoV-2 Omicron BA.1 variant: SARS-CoV-2 B.1.1.529 BA.1 strain isolated by the Shenzhen Center for Disease Control and Prevention (designated as SZ/08/2022/SARS-CoV-2(O)). SARS-CoV-2 Omicron BA.2 variant: SARS-CoV-2 B.1.1.529 BA.2 strain isolated by the Shenzhen Center for Disease Control and Prevention (designated as SARS-CoV-2/Shenzhen/13/2022 (Omicron BA.2)).
(1) Cell Preparation: One day prior to the experiment, VERO cells (1.5× 10⁴ cells/well) were seeded into a 96-well plate in a Biosafety Level-2 (BSL-2) laboratory using 10% FBS/DMEM culture medium.
(2) Virus Diluent Preparation: The SARS-CoV-2 Delta variant (titer: 1×10⁶ TCID50) and SARS-CoV-2 Omicron variant (titer: 1×10⁶ TCID50) were diluted to a final titer of 50 TCID50 using 2% FBS/DMEM. The diluted solutions were labeled as virus diluent Mix-D (Delta) and Mix-O (Omicron).
(3) Preparation of Sample-Virus Incubation Mixtures:
   (a) Nucleic Acid Sample-SARS-CoV-2 Delta Variant Mixtures:
      600 nM: 3.6 µl of 100 µM stock solution was mixed with 596.4 µl of Mix-D virus diluent.
      200 nM: 200 µl of 600 nM sample was mixed with 400 µl of Mix-D virus diluent.
      66.67 nM: 200 µl of 200 nM sample was mixed with 400 µl of Mix-D virus diluent.
      22.22 nM: 200 µl of 66.67 nM sample was mixed with 400 µl of Mix-D virus diluent.
      7.41 nM: 200 µl of 22.22 nM sample was mixed with 400 µl of Mix-D virus diluent.
      2.47 nM: 200 µl of 7.41 nM sample was mixed with 400 µl of Mix-D virus diluent.
      0.82 nM: 200 µl of 2.47 nM sample was mixed with 400 µl of Mix-D virus diluent.
      0.27 nM: 200 µl of 0.82 nM sample was mixed with 400 µl of Mix-D virus diluent.
      0.09 nM: 200 µl of 0.27 nM sample was mixed with 400 µl of Mix-D virus diluent.
      0 nM: 400 µl of Mix-D virus diluent was used without sample addition.
   (b) Nucleic Acid Sample-SARS-CoV-2 Omicron Variant Mixtures:
      600 nM: 3.6 µl of 100 µM stock solution was mixed with 596.4 µl of Mix-O virus diluent.
      200 nM: 200 µl of 600 nM sample was mixed with 400 µl of Mix-O virus diluent.
      66.67 nM: 200 µl of 200 nM sample was mixed with 400 µl of Mix-O virus diluent.
      22.22 nM: 200 µl of 66.67 nM sample was mixed with 400 µl of Mix-O virus diluent.
      7.41 nM: 200 µl of 22.22 nM sample was mixed with 400 µl of Mix-O virus diluent.
      2.47 nM: 200 µl of 7.41 nM sample was mixed with 400 µl of Mix-O virus diluent.
      0.82 nM: 200 µl of 2.47 nM sample was mixed with 400 µl of Mix-O virus diluent.
      0.27 nM: 200 µl of 0.82 nM sample was mixed with 400 µl of Mix-O virus diluent.
      0.09 nM: 200 µl of 0.27 nM sample was mixed with 400 µl of Mix-O virus diluent.
      0 nM: 400 µl of Mix-O virus diluent was used without sample addition.
(4) Incubation of Sample-Virus Mixtures: The serially diluted sample-virus mixtures were incubated in a 37°C, 5% CO₂ cell culture incubator for 1 hour.
(5) Loading and Incubation: The cell culture medium in the 96-well plate was aspirated. Subsequently, 100 µl of each sample-virus mixture was added to the corresponding labeled wells and incubated in a 37°C, 5% CO₂ incubator for 2 hours. Following incubation, the medium in each well was replaced with 120 µl of fresh 2% FBS/DMEM, and the plate was further incubated in a 37°C, 5% CO₂ incubator for 48 hours.
(6) Supernatant Nucleic Acid Extraction at 2 Days Post-Infection: After 48 hours, 100 µl of cell supernatant was collected into a 1.5 ml tube, and 200 µl of virus inactivation buffer (binding buffer from the kit) was added. The mixture was mixed thoroughly and incubated for 10 minutes. The samples were then transferred to a COVID-19 nucleic acid testing laboratory, where RNA extraction was performed using a Roche automated nucleic acid extraction system. The extracted RNA was adjusted to a final volume of 100 µl per sample, aliquoted for storage to avoid repeated freeze-thaw cycles during subsequent use. After supernatant collection, 100 µl of fresh 2% FBS/DMEM medium was replenished to each well.
(7) Supernatant Nucleic Acid Extraction at 5 Days Post-Infection: At 5 days post-infection, 100 µl of cell supernatant was collected into a 1.5 ml tube, and 200 µl of virus inactivation buffer (binding buffer from the kit) was added. The mixture was mixed thoroughly and incubated for at least 10 minutes before being transferred to the COVID-19 nucleic acid testing laboratory. RNA extraction was conducted using the Roche automated nucleic acid extraction system. The extracted RNA was adjusted to a final volume of 100 µl per sample, aliquoted for storage to prevent repeated freeze-thaw cycles.

RNA Extraction: In this experiment, RNA extraction was primarily performed on cell supernatants collected at different time points following infection with SARS-CoV-2 Delta, Omicron BA.1, and Omicron BA.2 variants. The RNA extraction from cell supernatants was conducted in a COVID-19 nucleic acid testing laboratory using the Roche MagNA Pure 96 automated nucleic acid extraction system for batch processing. Cell supernatants containing viruses were treated with virus inactivation reagent and disinfected with ethanol before removal from the Biosafety Level-3 (BSL-3) laboratory. All procedures in the COVID-19 nucleic acid testing laboratory were performed in compliance with regulations, and experimental waste was disposed of according to biosafety protocols.

One-Step RT-qPCR Assay: The extracted RNA was subjected to a one-step reverse transcription quantitative polymerase chain reaction (RT-qPCR) assay to quantify viral RNA expression, thereby evaluating the neutralizing inhibitory effects of the nucleic acid-based drug on viral replication.

### (1) Standard Curve Preparation

(a) Standard Reconstitution: The lyophilized SARS-CoV-2 nucleic acid N gene standard (5×10⁵ copies/vial) was centrifuged at 12,000 rpm for 2 minutes. Subsequently, 500 µl of DEPC-treated water was added to reconstitute the standard, achieving a final concentration of 1×10⁶ copies/ml.
(b) Standard Dilution: The reconstituted standard was serially diluted with DEPC-treated water to prepare standard solutions at concentrations of 1,000 copies/µl, 200 copies/µl, 40 copies/µl, 8 copies/µl, and 1.6 copies/µl. RNase-free pipette tips and tubes were used throughout the process, and all procedures were performed in a biosafety cabinet.
(c) Reagent Preparation: RNase-free pipette tips and tubes were utilized, and operations were conducted in a biosafety cabinet. The fluorescent PCR reaction mixture was prepared according to the following table.

**Table 6. Fluorescence PCR Reaction System**

| Reagents | Mass/Volume |
|---|---|
| Nucleic acid amplification reaction solution | 12 µl |
| Enzyme mixture | 4 µl |
| ORF1ab/N Reaction solution | 4 µl |
| Standard Dilution | 5 µl |

(d) Fluorescence quantitative PCR amplification: the fluorescence PCR reaction program was performed as per the table below.

**Table 7. Fluorescence PCR Amplification Procedures**

| Procedures | Reaction Time | |
|---|---|---|
| reverse transcription | 50°C, 10 min | |
| Initial denaturation | 95°C, 5 min | |
| Denaturation | 95°C, 10 s | 45 cycles |
| Annealing/Extension/ fluorescence Detection | 55°C, 40s | |

The fluorescence channels were configured as follows: FAM reporter for ORF1ab target detection and VIC/HEX for N target detection.

(e) Standard Curve Preparation: After the quantitative fluorescent PCR run was completed, the analysis was initiated, and the experimental data were exported. The CT values corresponding to each concentration were tabulated in Excel, with concentrations converted to logarithmic values (Log). The data was selected, and a scatter plot was generated by clicking "Insert" and selecting "Scatter Chart." The data points in the scatter plot were right-clicked, and a trendline was added. The trendline formatting was adjusted to display the equation and R-squared value. Axes, titles, and legends were appropriately labeled. An R-squared value greater than 0.99 indicated acceptable experimental data. In this experiment, the R² value was 0.9975, yielding the linear equation: Y = -3.7149X + 37.122, where X represents the logarithmic value of the sample concentration (copies/µl) and Y corresponds to the experimental CT value.

### (2) Experimental Sample Quantification

(a) Reaction Reagent Preparation: RNase-free pipette tips and tubes were used throughout the procedure, and all operations were conducted in a biosafety cabinet. Aliquoted RNA samples were retrieved from the -80°C freezer and thawed on ice. The fluorescent PCR reaction mixture was prepared as described in the standard curve preparation protocol.
(b) Fluorescent qPCR Amplification: The reaction program was configured according to the aforementioned amplification protocol. Since the N gene standard was used for the standard curve, data collection was primarily focused on the N detection target.
(c) Data Processing: After the fluorescent qPCR run was completed, the data were analyzed and exported. The CT values of each sample were substituted into the equation to calculate the corresponding sample concentration. The viral copy number was determined by multiplying the concentration by the sample volume. Relative copy numbers were calculated as (experimental group / average of control group) x 100%.

### Animal Experiments

All virus-related experimental procedures were conducted in a Biosafety Level-3 (BSL-3) laboratory at the Guangzhou Customs Technology Center, strictly adhering to BSL-3 operational protocols. SARS-CoV-2 Omicron BA.2 live virus has been reported to infect wild-type BALB/c mice. Preventive Efficacy Validation: Specific pathogen-free (SPF) 5-6-week-old BALB/c mice were lightly anesthetized with isoflurane and intranasally administered 1.6 mg/kg of the modified aptamer (Chol-PEG40K-5-2-15-2'-F-O), dissolved in sterile nuclease-free water (25 µl per nostril). Three hours post-administration, the mice were re-anesthetized with isoflurane and intranasally inoculated with 1×10⁵ focus-forming units (FFU) of Omicron BA.2 live virus. At 24/48 hours post-infection, the mice were euthanized under anesthesia. Lung tissues were macroscopically examined, harvested, and homogenized in PBS. Clear supernatants were collected, and RNA was extracted for RT-qPCR to quantify viral RNA. Viral titers per gram of lung tissue were additionally determined in VERO E6 cells using a focus reduction neutralization test (FRNT). Therapeutic Efficacy Validation: SPF 5-6-week-old BALB/c mice were lightly anesthetized with isoflurane and intranasally inoculated with 1×10⁵ FFU of Omicron BA.2 live virus. Three hours post-infection, the mice were re-anesthetized and intranasally administered 1.6 mg/kg of the modified aptamer (Chol-PEG40K-5-2-15-2'-F-O), dissolved in sterile nuclease-free water (25 µl per nostril). At 24/48 hours post-treatment, the mice were euthanized under anesthesia. Lung tissues were macroscopically examined, harvested, and homogenized in PBS. Clear supernatants were collected, and RNA was extracted for RT-qPCR to quantify viral RNA. Viral titers per gram of lung tissue were similarly assessed in VERO E6 cells via FRNT.

### Statistical Methods and Data Analysis

All data in this study were derived from three or more independent replicates and are presented as mean ± standard deviation. Microsoft Excel 2021 was used for statistical calculations, and GraphPad Prism 9 was employed for data processing and graphical representation. Statistical significance was analyzed using a two-tailed Student's t-test, with the following notations: * for t<0.05, ** for t<0.01, *** for t<0.001, **** for t<0.0001 and NS (not significant) for nonsignificant differences.

### Sequences

The amino acid and nucleic acid sequences described in this invention are provided in the table below.

**Table 8. Sequences involved in the present invention.**

| **Sequence ID** | **Description** | **Sequences** |
|---|---|---|
| SEQ ID NO: 1 | #2-1-17 random region | CUCGGUAUCUAGUGCGCACU |
| SEQ ID NO: 2 | #2-1-18 random region | CGUCGACUGAAAUCCUCUUC |
| SEQ ID NO: 3 | #2-2-24 random region | UGUUCGUUUUCGCCCCGUGG |
| SEQ ID NO: 4 | #3-1-31 random region | AUAGUGAAUACUACAGAGAC |
| SEQ ID NO: 5 | #3-2-3 random region | UCUUACAAUGUUUAGAAGGU |
| SEQ ID NO: 6 | #4-1-20 random region | UGUACAGAAUUCUCCCGGGU |
| SEQ ID NO: 7 | #5-2-15 random region | AUUACCGUAGUUUUUGUAGU |
| SEQ ID NO: 8 | 5' end constant sequence | ACUUGGCCA |
| SEQ ID NO: 9 | 3' end constant sequence | CUGCAGGGCCAAGUG |
| SEQ ID NO: 10 | Oligo Random Library | AGCAAGTTAAAATAAGGCTAGTCCGTTATCAACTT GGCCAnnnnnnnnnnnnnnnnnnnnCTGCAGGGCCAAGTG GCACCGAGTCGGTGCTTTTTATCGA |
| SEQ ID NO: 11 | oligo-Primer F | TATGTTTAAGAGCTAGAAATAGCAAGTTAAAATAA GGCTA |
| SEQ ID NO: 12 | oligo-Primer R | CCATCTTTGCAAAGCTTATATCGATAAAAAGCACCG ACTC |
| SEQ ID NO: 13 | #2-1-17 full sequence | ACUUGGCCACUCGGUAUCUAGUGCGCACUCUGCA GGGCCAAGUG |
| SEQ ID NO: 14 | #2-1-18 full sequence | ACUUGGCCACGUCGACUGAAAUCCUCUUCCUGCA GGGCCAAGUG |
| SEQ ID NO: 15 | #2-2-24 full sequence | ACUUGGCCAUGUUCGUUUUCGCCCCGUGGCUGCA GGGCCAAGUG |
| SEQ ID NO: 16 | #3-1-31 full sequence | ACUUGGCCAAUAGUGAAUACUACAGAGACCUGC AGGGCCAAGUG |
| SEQ ID NO: 17 | #3-2-3 full sequence | ACUUGGCCAUCUUACAAUGUUUAGAAGGUCUGC AGGGCCAAGUG |
| SEQ ID NO: 18 | #4-1-20 full sequence | ACUUGGCCAUGUACAGAAUUCUCCCGGGUCUGC AGGGCCAAGUG |
| SEQ ID NO: 19 | #5-2-15 full sequence | ACUUGGCCAAUUACCGUAGUUUUUGUAGUCUGC AGGGCCAAGUG |
| SEQ ID NO: 20 | Canonical GFP aptamer | GCUUCUGGACUGCGAUGGGAGCACGAAACGUCG UGGCGCAAUUGGGUGGGGAAAGUCCUUAAAAGA GGGCCACCACAGAAGC |
| SEQ ID NO: 21 | hU6-gLuc sgRNA-1.2 BsmBI-BsmBI backbone | TTTCCCATGATTCCTTCATATTTGCATATACGATACA AGGCTGTTAGAGAGATAATTAGAATTAATTTGACTG TAAACACAAAGATATTAGTACAAAATACGTGACGT AGAAAGTAATAATTTCTTGGGTAGTTTGCAGTTTTA AAATTATGTTTTAAAATGGACTATCATATGCTTACCG TAACTTGAAAGTATTTCGATTTCTTGGCTTTATATAT CTTGTGGAAAGGACGAAACACCGATCTAGATACGA CTCACTATGTTTAAGAGCTAGAAATAGCAAGTTAA AATAAGGCTAGTCCGTTATCAACTTGGCCAGGAGA CGGACGTCTCACTGCAGGGCCAAGTGGCACCGAG TCGGTGCTTTTT |
| SEQ ID NO: 22 | RBD-VPH | RVQPTESIVRFPNITNLCPFGEVFNATRFASVYAWNR KRISNCVADYSVLYNSASFSTFKCYGVSPTKLNDLCF |
| | | TNVYADSFVIRGDEVRQIAPGQTGKIADYNYKLPDD FTGCVIAWNSNNLDSKVGGNYNYLYRLFRKSNLKPF ERDISTEIYQAGSTPCNGVEGFNCYFPLQSYGFQPTN GVGYQPYRVVVLSFELLHAPATVCGPKKSTNLVKNK CVNFEFGGSGGSGGSDRGSDALDDFDLDMLGSDAL DDFDLDMLGSDALDDFDLDMLGSDALDDFDLDML GSSGGGGGSAINSQYLPDTDDRHRIEEKRKRTYETFK SIMKKSPFSGPTDPRPPPRRIAVPSRSSASVPKPAPQPY PFTSSLSTINYDEFPTMVFPSGQISQASALAPAPPQVL PQAPAPAPAPAMVSALAQAPAPVPVLAPGPPQAVAPP APKPTQAGEGTLSEALLQLQFDDEDLGALLGNSTDP AVFTDLASVDNSEFQQLLNQGIPVAPHTTEPMLMEY PEAITRLVTGAQRPPDPAPAPLGAPGLPNGLLSGDED FSSIADMDFSALLSGGGGGSGFSVDTSALLDLFSPSV TVPDMSLPDLDSSLASIQELLSPQEPPRPPEAENSSPD SGKQLVHYTAQPLFLLDPGSVDTGSNDLPVLFELGE GSYFSEGDGFAEDPTISLLTGSEPPKAKDPTVS (linker is underlined) |
| SEQ ID NO: 23 | dCas9 | MDKKYSIGLAIGTNSVGWAVITDEYKVPSKKFKVLG NTDRHSIKKNLIGALLFDSGETAEATRLKRTARRRYT RRKNRICYLQEIFSNEMAKVDDSFFHRLEESFLVEED KKHERHPIFGNIVDEVAYHEKYPTIYHLRKKLVDSTD KADLRLIYLALAHMIKFRGHFLIEGDLNPDNSDVDK LFIQLVQTYNQLFEENPINASGVDAKAILSARLSKSR RLENLIAQLPGEKKNGLFGNLIALSLGLTPNFKSNFD LAEDAKLQLSKDTYDDDLDNLLAQIGDQYADLFLA AKNLSDAILLSDILRVNTEITKAPLSASMIKRYDEHH QDLTLLKALVRQQLPEKYKEIFFDQSKNGYAGYIDG GASQEEFYKFIKPILEKMDGTEELLVKLNREDLLRKQ RTFDNGSIPHQIHLGELHAILRRQEDFYPFLKDNREKI EKILTFRIPYYVGPLARGNSRFAWMTRKSEETITPWN FEEVVDKGASAQSFIERMTNFDKNLPNEKVLPKHSL LYEYFTVYNELTKVKYVTEGMRKPAFLSGEQKKAIV DLLFKTNRKVTVKQLKEDYFKKIECFDSVEISGVEDR FNASLGTYHDLLKIIKDKDFLDNEENEDILEDIVLTLT LFEDREMIEERLKTYAHLFDDKVMKQLKRRRYTGW GRLSRKLINGIRDKQSGKTILDFLKSDGFANRNFMQL IHDDSLTFKEDIQKAQVSGQGDSLHEHIANLAGSPAI KKGILQTVKVVDELVKVMGRHKPENIVIEMARENQT TQKGQKNSRERMKRIEEGIKELGSQILKEHPVENTQL QNEKLYLYYLQNGRDMYVDQELDINRLSDYDVDAI VPQSFLKDDSIDNKVLTRSDKNRGKSDNVPSEEVVK KMKNYWRQLLNAKLITQRKFDNLTKAERGGLSELD KAGFIKRQLVETRQITKHVAQILDSRMNTKYDENDK LIREVKVITLKSKLVSDFRKDFQFYKVREINNYHHAH DAYLNAVVGTALIKKYPKLESEFVYGDYKVYDVRK MIAKSEQEIGKATAKYFFYSNIMNFFKTEITLANGEIR KRPLIETNGETGEIVWDKGRDFATVRKVLSMPQVNI VKKTEVQTGGFSKESILPKRNSDKLIARKKDWDPKK YGGFDSPTVAYSVLVVAKVEKGKSKKLKSVKELLGI TIMERSSFEKNPIDFLEAKGYKEVKKDLIIKLPKYSLF ELENGRKRMLASAGELQKGNELALPSKYVNFLYLAS HYEKLKGSPEDNEQKQLFVEQHKHYLDEIIEQISEFS KRVILADANLDKVLSAYNKHRDKPIREQAENIIHLFT LTNLGAPAAFKYFDTTIDRKRYTSTKEVLDATLIHQSI TGLYETRIDLSQLGGDEGA |
| SEQ ID NO: 24 | dCasMINI-V4 | MAKNTITKTLKLRIVRPYNSAEVEKIVADEKNNREKI ALEKNKDKVKEACSKHLKVAAYCTTQVERNACLFC KARKLDDKFYQKLRGQFPDAVFWQEISEIFRQLQKQ AAEIYNQSLIELYYEIFIKGKGIANASSVEHYLSRVCY RRAAELFKNAAIASGLRSKIKSNFRLKELKNMKSGLP TTKSDNFPIPLVKQKGGQYTGFEISNHNSDFIIKIPFGR WQVKKEIDKYRPWEKFDFEQVQKSPKPISLLLSTQR RKRNKGWSKDEGTEAEIKKVMNGDYQTSYIEVKRG SKICEKSAWMLNLSIDVPKIDKGVDPSIIGGIAVGVRS PLVCAINNAFSRYSISDNDLFHFNKKMFARRRILLKK NRHKRAGHGAKNKLKPITILTEKSERFRKKLIERWAC EIADFFIKNKVGTVQMENLESMKRKEDSYFNIRLRGF WPYAEMQNKIEFKLKQYGIEIRKVAPNNTSKTCSKC GHLNNYFNFEYRKKNKFPHFKCEKCNFKENAAYNA ALNISNPKLKSTKERPA |
| SEQ ID NO: 25 | S/RBD-Primer F | GGCGGTAGTGGTGGTGAATTCAGAGTCCAACCAAC AGAATCT |
| SEQ ID NO: 26 | S/RBD-Primer R | ACCGCCAGAGCCACCGAATTCGAAATTGACACATT TGTTTTTAACC |
| SEQ ID NO: 27 | mini-promoter-2 | TAGAGGGTATATAATGGAAGCTCGACTTCCAG |
| SEQ ID NO: 28 | mini-promoter-1 | TAGAGGGTATATAATGGAAGCTCGAATTCCAG |
| SEQ ID NO: 29 | mini-TK promoter | TTCGCATATTAAGGTGACGCGTGTGGCCTCGAACA CCGAGCGACCCTGCAGCGACCCGCTTAA |
| SEQ ID NO: 30 | mini-CMV promoter | GGTAGGCGTGTACGGTGGGAGGTCTATATAAGCAG AGCT |
| SEQ ID NO: 31 | Crystallin basal promoter | AGTAGAAGCTCTCGATCGCGAATCTGTTCCCGGGG ATCCTCTAGAGTCGACCTGCAGGGCCAGCCCAGGC TCCGGGGCACGTAAAAGCGGGGCTGTGAGACCGG AGAGCACGGAGCGACCAGCCAGGGCTGAGCTGCG GAGACGGTGAGCAGGGCTGTGCGGGCAGCCAAGC TTGGCATTCCGGTACTGTTGGTAAA |
| SEQ ID NO: 32 | gLuc target | ATCTAGATACGACTCACTATAGG (PAM is underlined) |
| SEQ ID NO: 33 | 9x gLuc target | ATCTAGATACGACTCACTATAGGcaaagctctaATCTAGA TACGACTCACTATAGGaaaggaagcagccacATCTAGATAC GACTCACTATAGGataacgttagATCTAGATACGACTCAC TATAGGgtgaagtagtctttgcggtaggagacctattcgagATCTAGAT ACGACTCACTATAGGcaaagctctaATCTAGATACGACT CACTATAGGaaaggaagcagccacATCTAGATACGACTCA CTATAGGataacgttagATCTAGATACGACTCACTATAGG gtgaagtagtctttgcggtaATCTAGATACGACTCACTATAGGat ccacgtatgtcgagggt |
| SEQ ID NO: 34 | Tet target | tttgCTCCCTATCAGTGATAGAGAACG (PAM is underlined) |
| SEQ ID NO: 35 | 9x Tet target | tttgCTCCCTATCAGTGATAGAGAACGcaaagctctatttgCT CCCTATCAGTGATAGAGAACGaaaggaagcagccactttgCT CCCTATCAGTGATAGAGAACGataacgttagtttgCTCCCT ATCAGTGATAGAGAACGgtgaagtagtctttgcggtatttgCTCC CTATCAGTGATAGAGAACGcaaagctctatttgCTCCCTATC AGTGATAGAGAACGaaaggaagcagccactttgCTCCCTATC AGTGATAGAGAACGataacgttagtttgCTCCCTATCAGTG ATAGAGAACGgtgaagtagtctttgcggtatttgCTCCCTATCAG TGATAGAGAACG |

### Example 1. Design of the Nucleic Acid Aptamer Screening System

As illustrated in Figure 1A, the nucleic acid aptamer screening system of this invention utilized the guide function of sgRNA and the targeting capability of dCas9 to anchor the dCas9-sgRNA complex to the sgRNA-targeted sequence upstream of a minimal promoter (mini-promoter-2, SEQ ID NO: 27). Simultaneously, the target protein of interest (X-protein) was fused with transcriptional activation modules. If the aptamer inserted into the loop region of the sgRNA scaffold interacts with the freely expressed X-protein via affinity recognition, the transcription activation modules fused to X-protein are recruited to the sgRNA-targeted sequence, thereby activating the expression of the downstream selection marker gene under mini-promoter-2. The expression of the downstream marker gene indirectly reflects the affinity-binding capacity between the aptamer and the target protein.

Previous studies have demonstrated that extensive base substitutions or deletions in sgRNA scaffold regions, such as stem loop 2 and tetra loop, do not impair the catalytic function of Cas9. These loop regions could tolerate the insertion of aptamers to enhance the recruitment of Cas9 or its effector complexes. Additionally, the sgRNA scaffold allows large-fragment insertions without compromising dCas9 activity.

### Screening Process

As illustrated in Figure 1B, the screening process was initiated by cloning synthetic random oligonucleotide sequences into the loop region of a customized lentiviral vector sgRNA scaffold through sgRNA library construction, thereby generating an initial library containing sgRNA-targeted exogenous sequences with appended random oligonucleotide sequences in the scaffold region. Notably, the customized lentiviral vector was pre-engineered to include independent open reading frames (ORFs) for co-expression of X-protein with transcriptional activation modules, enabling facile molecular cloning replacement of X-protein. Subsequently, the library was subjected to lentiviral packaging and transduction into a universal screening reporter cell line.

Critically, the universal screening reporter cell line was pre-integrated with a selection reporter system (sgRNA-targeted sequence-mini-promoter-2-selection marker gene) that exhibited sensitivity to selection pressure. Due to the integrative nature of lentiviral vectors, DNA sequences encoding the random oligonucleotides were stably incorporated into the genome of the reporter cell line. If a nucleic acid aptamer with affinity for X-protein was present within the random oligonucleotide sequences inserted into the sgRNA scaffold, the co-expressed transcriptional activation modules fused to X-protein were recruited to the sgRNA-targeted sequence (i.e., the upstream region of the screening reporter system).

When selection pressure corresponding to the marker gene was applied, the reporter system in the cell line was activated. The stronger the affinity between the aptamer and X-protein, the greater the recruitment of transcriptional activation modules, leading to enhanced activation of the downstream selection marker gene and a more robust cellular response to selection pressure. Following screening, genomic DNA from enriched cells was extracted, and the aptamer sequences inserted into the sgRNA scaffold loop region were amplified. These sequences were analyzed via next-generation sequencing (NGS) (Figure 1B) to identify aptamers with high affinity for the target protein.

### Example 2. Validation of the Nucleic Acid Aptamer Screening System

The system was validated using a luciferase reporter system, where the luciferase reporter gene was positioned downstream of mini-promoter-2. The guide function of sgRNA and the targeting capability of dCas9 ensured that the dCas9-sgRNA complex was anchored to the sgRNA-targeted sequence. If the aptamer embedded in the sgRNA scaffold was capable of affinity recognition for the target protein, the CRISPRa (transcriptional activation modules) co-expressed with the target protein were recruited to the sgRNA-targeted sequence. Subsequently, the transcriptional activation modules acted on mini-promoter-2 to activate the expression of the luciferase reporter gene (Figure 2B). The expression of the downstream luciferase reporter gene was dependent from the efficiency of aptamer-mediated recruitment of the target protein-CRISPRa complex, thereby transducing the affinity interaction between the aptamer and the target protein into measurable expression of luciferase reporter gene.

The canonical GFP aptamer, which was identified through the SELEX screening system targeting Green Fluorescent Protein (GFP), was utilized to validate that transcription activators could be recruited, and luciferase reporter gene expression could be activated in cells via the affinity interaction between RNA aptamers and target proteins.

First, the canonical GFP aptamer was inserted into either the tetra loop region (sgRNA 1.1) or the stem loop2 region (sgRNA 1.2) of the sgRNA scaffold (Figure 2A). Concurrently, GFP was fused with the transcriptional activation elements VP64, P65, and HSF1 (abbreviated as VPH) for co-expression (Figure 2B). A luciferase reporter plasmid containing one copy of the gLuc-targeting sequence (1×gLuc-minPro-luciferase) was constructed. Subsequently, the 293T/dCas9 cell line stably expressing dCas9 was co-transfected with three plasmids: (1) either the sgRNA1.1 or sgRNA1.2 construct, (2) GFP-VPH, and (3) 1×gLuc-minPro-luciferase. After 48 hours, the luciferase expression signal was detected.

As shown in Figure 2C, the reporter gene expression capability was not significantly altered in either the sgRNA 1.1 group or the sgRNA 1.2 group, indicating that the insertion of the canonical GFP aptamer into the tetra loop region (sgRNA 1.1) or stem loop2 region (sgRNA 1.2) of the sgRNA scaffold did not affect dCas9 activity.

To further investigate whether the developed system exhibited a cumulative effect, a luciferase reporter plasmid containing nine copies of the gLuc-targeting sequence (9×gLuc-minPro-luciferase) was constructed. The 293T/dCas9 cell line stably expressing dCas9 was co-transfected with three plasmids: (1) either the sgRNA1.1 or sgRNA1.2 construct, (2) GFP-VPH, and (3) either 1xgLuc-minPro-luciferase or 9xgLuc-minPro-luciferase. The luciferase expression signal was detected 48 hours post-transfection.

The results were shown in Figures 2C and 2D. Compared to the single-copy gLuc sgRNA target sequence (Figure 2C), a significant luciferase expression signal was detected when nine tandem repeats of the gLuc sgRNA target sequence were employed. This indicated that the utilization of multiple copies of the gLuc target sequence facilitated the recruitment of a greater number of CRISPRa transcriptional activation modules, thereby enhancing the sensitivity of the system (Figure 2D). Furthermore, compared to the GFP aptamer appended to the tetraloop region of the sgRNA scaffold (sgRNA 1.1), the GFP aptamer appended to the stem loop2 region of the sgRNA scaffold (sgRNA 1.2) demonstrated a more pronounced luciferase activation effect.

### Example 3. Construction of a Universal Screening Cell Line

To facilitate the collection of successfully enriched cells under selective pressure post-screening, the reporter gene was replaced by a puromycin resistance gene. To enhance the operability and convenience of subsequent system screening, more 293T monoclonal cell lines co-expressing dCas9 and 9×gLuc-puro were obtained via delivery of lentiviral reporter system components (9×gLuc-minPro-puro) into the 293T/dCas9 monoclonal cell line.

To validate the efficacy and background activity of these monoclonal cell lines, the affinity relationship between GFP and the GFP aptamer was utilized for verification. These monoclonal cell lines were transduced with the following lentiviral components: Positive Controls (PC): gLuc sgRNA-1.2 GFP aptamer+ GFP-VPH, Negative Controls (NC): gLuc sgRNA-1.2 random sequence+GFP-VPH (Figure 3). Forty-eight hours post-lentiviral transduction, varying concentrations of puromycin (0.5 µg/ml, 1 µg/ml, and 2 µg/ml) were applied to distinct experimental groups of these monoclonal cell lines, with increasing concentrations representing escalating selection pressure. After 7-10 days of continuous puromycin treatment, the number of surviving colonies in each dCas9/9×gLuc-puro monoclonal cell line was quantified (Figure 4).

Monoclonal cell lines with fewer surviving colonies after transduction with NC viral components were defined as those exhibiting lower background activity, while those with higher numbers of surviving colonies after transduction with PC viral components were classified as cell lines demonstrating sensitivity to the screening system and robust survival capacity. Monoclonal cell lines with elevated PC/NC ratios were identified as those possessing superior comprehensive activity.

Quantitative results were shown in Figure 4. Compared to other puromycin concentrations, the majority of monoclonal cell lines demonstrated higher PC/NC ratios at 2 µg/ml puromycin, indicating that elevated puromycin concentrations could effectively filter out background activity. Furthermore, across varying puromycin concentrations, clone #4-53 consistently exhibited a high PC/NC ratio. Notably, under 2 µg/ml puromycin treatment, this cell line displayed extremely low background activity coupled with high survival capacity (Figure 2E). Consequently, clone #4-53 was selected as the universal screening cell line for subsequent nucleic acid aptamer screening.

### Example 4. Construction and Screening Process of a Universal Nucleic Acid Aptamer Screening System

Based on the validation results of the CRISPR/Cas9-based RNA aptamer screening system described above, a universal RNA aptamer screening system utilizing the CRISPR/Cas9 system was proposed and named CRISmers. When applied for RNA aptamer screening, the CRISmers system demonstrated dual advantages: (1) the affinity recognition capability between aptamers and target proteins was quantitatively converted within cells, ensuring the maximal preservation of the native structural state of the target proteins while maintaining environmental authenticity for future aptamer functionality; and (2) multiple rounds of screening combined with high-concentration antibiotic selection in each round effectively minimized background noise caused by dead cells and enhanced screening stringency.

To streamline library construction, luciferase reporter system validation, and experimental operability, a customizable lentiviral vector was constructed: phU6-gLuc sgRNA-1.2 BsmBI-BsmBI-polyT-EF1a-NLS-linker-EcoRI-EcoRI-linker-VP64-P65-HSF1-NLS-linker-P2A-linker-Zeo-WPRE (Figure 12). Key features included: (1) a BsmBI restriction site for inserting the gLuc sgRNA-1.2 structural region; (2) an EcoRI restriction site for inserting the target protein of interest; and (3) fusion expression of the target protein with the transcriptional activation module VPH. This lentiviral vector enabled single-step molecular cloning for replacing target proteins of interest and required only one library construction to generate an oligonucleotide random sequence library specific to the target protein. To support library screening, the universal screening monoclonal cell line #4-53 dCas9/9×gLuc-puro, constructed in Example 3, was utilized.

As shown in Figure 5, the workflow of a screening cycle was divided into the following seven steps: First, the EcoRI restriction endonuclease was utilized to insert a custom target protein of interest into the universal lentiviral expression vector, forming an intermediate vector. An oligonucleotide random sequence library was synthesized and cloned into the stem-loop2 region of the gLuc sgRNA backbone in the intermediate vector using the BsmBI restriction endonuclease, thereby completing the library construction. Second, the aforementioned library, expressing oligonucleotide random sequences targeting the specific protein for screening, was packaged into lentiviral particles. The viral titer was quantified using Zeocin antibiotic selection, completing the lentiviral library packaging. Third, these lentiviral particles were used to infect the #4-53 dCas9/9×gLuc-puro universal screening reporter monoclonal cell line at a defined multiplicity of infection (MOI). Fourth, 2 µg/ml puromycin selection pressure was applied to the lentivirus-infected cells as the screening progressed. Fifth, surviving cells were collected following puromycin-based enrichment. Sixth, cells were lysed, and DNA sequences containing the aptamer region were specifically amplified. The obtained sequences were analyzed and validated via deep sequencing. Identified aptamer candidates were subjected to secondary screening and validation using the luciferase reporter system. Seventh, if necessary, the amplified aptamer sequences from the previous round were subcloned into the original universal lentiviral expression vector to initiate subsequent rounds of screening.

### Example 5. Screening of RNAAptamers Targeting the Receptor-Binding Domain (RBD) of the SARS-CoV-2 Spike Protein

Given the urgent need for SARS-CoV-2 diagnostic and therapeutic strategies, the CRISmers system was utilized to screen RNA aptamers targeting the RBD region of the SARS-CoV-2 spike (S) protein. In the experiment, two independent parallel screenings were performed, each consisting of five rounds. In the first round of screening, to maximize the representation of aptamers in infected cells, a lentiviral library containing 20-nucleotide randomized sequences appended to the stem-loop2 region of the gLuc sgRNA backbone and targeting the RBD of the S1 protein was used to infect the #4-53 9×gLuc-puro universal screening reporter monoclonal cell line at a multiplicity of infection (MOI) of ~3. From the second round onward, the sublibraries were infected into the same cell line at a lower MOI (0.1-0.2) to ensure that most cells were transduced with only one viral particle harboring a 20-nucleotide sequence.

Using the CRISmers system, RNA aptamers targeting the RBD region of the SARS-CoV-2 spike (S) protein were screened. Since aptamer lengths are typically concentrated between 20-40 nucleotides, an oligonucleotide random sequence library with 20-nucleotide diversity was synthesized. Due to technical limitations in molecular biology, the full combinatorial diversity (4²⁰ unique sequences) could not be achieved; however, efforts were maximized during library construction to generate as many oligonucleotide sequences as possible. Two independent parallel screening replicates were performed. During the initial screening round, cells were infected with the lentiviral library at a higher MOI to maximize the representation of aptamers in infected cells.

Through iterative cycles of oligonucleotide random library construction, lentiviral library packaging, lentiviral infection, 2 µg/ml puromycin selection, collection of surviving cells, sequence amplification and deep sequencing, and sublibrary construction, a total of five rounds of screening were completed for both independent replicates. Analysis of deep sequencing data from the second, third, fourth, and fifth rounds revealed that, after filtering out nonspecific sequences, the number of unique aptamer sequences gradually decreased as screening progressed (see Table below). By the fourth and fifth rounds, no significant reduction in unique aptamer sequences was observed, indicating that enrichment pressure was sufficient and the library had reached saturation, rendering further sublibrary screening unnecessary (Figure 6).

**Table 9. Sequence Analysis After Each Round**

| Rounds of Screening (Replicate 1) | Total Number of Sequences after filtering | Number of Unique Sequences | Rounds of Screening (Replicate 2) | Total Number of Sequences after filtering | Number of Unique Sequence |
|---|---|---|---|---|---|
| R1 (First Round) | 4155886 | 97585 | R1 (First Round) | 490172 | 103387 |
| R2 (Second Round) | 5961393 | 39162 | R2 (Second Round) | 419350 | 20807 |
| R3 (Third Round) | 4304426 | 27282 | R3 (Third Round) | 368900 | 16285 |
| R4 (Fourth Round) | 4696696 | 17712 | R4 (Fourth Round) | 433481 | 15796 |
| R5 (Fifth Round) | 6899951 | 10819 | R5 (Fifth Round) | 670683 | 13764 |

Next, the selected RNA aptamers were subjected to secondary screening and validation using the luciferase reporter system.

For the two independent screening replicates, sequences obtained from deep sequencing of the second, third, fourth, and fifth rounds were prioritized in two ways. First, frequency-based ranking: all unique sequences were ranked by their read counts, and the top 0.1% of high-frequency sequences were selected for validation. Second, enrichment index ranking: the enrichment index (ratio of read counts between consecutive rounds for each unique sequence) was calculated, and the top 15 sequences with the highest enrichment indices per round were selected for validation. The RNA aptamers prioritized by these two methods were first re-screened using the luciferase reporter system. The selected sequences were synthesized and cloned into the S1 RBD-targeting expression vector, phU6-gLuc sgRNA-1.2-BsmBI-BsmBI-polyT-EF1a-NLS-linker-S1 RBD-linker-VP64-P65-HSF1-NLS-linker-P2A-linker-Zeo-WPRE, using the BsmBI restriction endonuclease. In 293T cells, three plasmids were co-transfected: the 9xgLuc sgRNA-gaussia luciferase construct, the dCas9 expression cassette, and the aptamer-containing target vector. This experimental setup was designed to verify whether the selected RNA aptamers could recruit transcriptional activation modules via their affinity to the RBD and subsequently activate Gaussia luciferase expression.

It should be noted that during this secondary screening, firefly luciferase was replaced with secreted Gaussia luciferase, enabling direct detection of luciferase secreted into the cell supernatant. This modification streamlined experimental workflows and improved efficiency in high-throughput assays. Additionally, it was observed that high-frequency sequences could represent PCR bias-derived artifacts and might not necessarily exhibit high enrichment fold changes, indicating that frequency and enrichment were not strictly correlated. Furthermore, during sequence analysis in the fourth and fifth rounds, certain high-frequency or high-enrichment-index sequences were identified and validated in earlier screening rounds, confirming that sufficient screening pressure had been achieved by the fourth round. This observation aligned with the saturation trends observed in deep sequencing data, validating the consistency of the screening system. The selected RNA aptamer candidates were validated using the luciferase reporter system. As shown in Figure 7, aptamers with strong luciferase activation signals were detected as early as the second screening round. Aptamers #2-1-17, #2-1-18, #2-2-24, #3-1-31, #3-2-3, #4-1-20, and #5-2-15 exhibited statistically significant luciferase activation; their sequences are listed in Table 8.

To ensure accuracy, sequences showing consistent luciferase activation across multiple rounds were compared in parallel. In Figure 8A, aptamers #2-1-18 and #5-2-15 demonstrated the highest luciferase activation signals. Figure 8B illustrates the structural integration of these aptamers into the sgRNA backbone. Secondary structures and free energy values of the RNA aptamers, predicted using the Mfold webserver, are shown in Figure 8C.

### Example 6. Detection Capacity of RNA Aptamers Against SARS-CoV-2 and Variant Pseudoviruses

Aptamers #2-1-18 and #5-2-15 were selected for subsequent functional validation. First, affinity and specificity assays were performed using an enzyme-linked oligonucleotide assay (ELONA) (Figure 9A).

As shown in Figure 9B, both RNA aptamers exhibited high-affinity binding to the RBD recombinant protein. The equilibrium dissociation constants (Kd) for aptamers #2-1-18 and #5-2-15 were calculated to be 16.61 nM and 22.65 nM, respectively.

To evaluate diagnostic sensitivity, the detection limit of the RNA aptamers was examined by testing them against serially diluted RBD recombinant protein using 100 nM aptamers in the ELONA. Negative controls included the recombinant programmed cell death protein 1 (PD1), the SARS-CoV-2 non-structural protein 7 and 8 complex (NSP7-8), and random RNA sequences of equivalent length.

As shown in Figures 9C and 9D, both aptamers targeting the SARS-CoV-2 wild-type spike protein RBD recombinant protein demonstrated strong affinity and specificity, with detection limits as low as 0.1 µg/ml for the RBD recombinant protein. Similarly, in Figures 9D and 9H, both aptamers exhibited high affinity and specificity when tested against the RBD recombinant protein of the SARS-CoV-2 Delta variant of concern (VOC). In Figures 9E and 9I, the aptamers were shown to bind effectively to the RBD recombinant protein of the SARS-CoV-2 Omicron BA.1 VOC. Figures 9F and 9J further confirmed their robust binding to the RBD recombinant protein of the SARS-CoV-2 Omicron BA.2 VOC.

To assess the detection limits against pseudoviruses, the RNA aptamers (100 nM) were tested in the ELONA assay against serially diluted pseudovirus particles displaying: SARS-CoV-2 wild-type spike protein (Figure 9K), SARS-CoV-2 Delta VOC spike protein (Figure 9L), SARS-CoV-2 Omicron BA.1 VOC spike protein (Figure 9M), and SARS-CoV-2 Omicron BA.2 VOC spike protein (Figure 9N). Random RNA sequences of equivalent length were included as negative controls in all experiments.

As shown in Figures 9K-9N, both aptamers exhibited strong affinity and specificity, enabling detection of pseudovirus concentrations as low as 1 TCID50/100 µl (50% tissue culture infective dose) for all four pseudovirus variants.

Further analysis based on RBD recombinant protein ELONA assays (Figure 10) revealed that #core-5-2-15, with complementary constant flanking sequences at both ends, facilitated efficient sequence synthesis and amplification while significantly enhancing the aptamer's affinity for the target protein. During screening, random sequences were observed to form variable secondary structures via base-pairing interactions with the constant flanking sequences, potentially interfering with aptamer formation. Thus, in preferred embodiments, the full-length RNA aptamer sequence included both the screened functional core and the constant flanking sequences.

Additionally, as shown in Figure 11, in ELONA assays using RBD recombinant protein, both aptamers retained binding activity at 4°C, 25°C, and 37°C, indicating their potential utility across a broad range of working temperatures.

### Example 7. Neutralization Activity of RNA Aptamers Against Live SARS-CoV-2 Variants of Concern

Given that the binding of RNA aptamers to the RBD might block the interaction between the SARS-CoV-2 spike protein and human ACE2 (hACE2), neutralization activity assays were performed in a BSL-3 laboratory to evaluate the inhibitory effects of the #5-2-15 RNA aptamer against live SARS-CoV-2 variants of concern (VOCs): Delta (B.1.617.2) and Omicron BA.1 (B.1.1.529 BA.1). The experimental workflow is illustrated in Figure 12A. Aptamer #5-2-15 was prioritized for validation due to its superior binding activity observed in prior experiments.

Prior to infecting VERO cells with live SARS-CoV-2 virus, varying concentrations of the RNA aptamer were pre-incubated with live virus particles. The aptamer-virus mixture was then incubated with VERO cells. Cell supernatants were collected at 2 and 4 days post-infection, and viral RNA was extracted. Viral copy numbers were quantified using RT-qPCR to assess neutralization efficacy.

The results demonstrated that RNA aptamer #5-2-15 exhibited dose-dependent inhibitory activity against both SARS-CoV-2 VOCs at two distinct time points, consistent with its binding affinity to pseudoviruses (Figures 9H and 9I). As shown in Figures 12B and 12C, at 2 days post-infection, the half-maximal inhibitory concentration (IC50) of #5-2-15 against SARS-CoV-2 Delta was calculated to be 26.25 ng/ml, while its IC50 against SARS-CoV-2 Omicron BA.1 was 0.4628 ng/ml. At 4 days post-infection (Figures 12D and 12E), the IC50 values for #5-2-15 against Delta and Omicron BA.1 were 19.91 ng/ml and 0.6006 ng/ml, respectively.

Additionally, the antiviral activity of the alternative aptamer #2-1-18 against Omicron BA.1 was evaluated. As shown in Figures 13A and 13B, the IC50 values for #2-1-18 against Omicron BA.1 were determined to be 6.732 ng/ml at 2 days post-infection and 10.23 ng/ml at 4 days post-infection.

### Example 8. Neutralization Activity of Chemically Modified RNA Aptamers Against Live SARS-CoV-2 Variants of Concern

Chemical modifications were introduced to the selected RNA aptamers to enhance nucleotide stability, prolong half-life, and reduce immunogenicity. The binding activity and specificity of unmodified versus modified aptamers were evaluated using ELONA and electrophoretic mobility shift assay (EMSA).

As shown in Figures 14A-14B, fluorination and methoxy co-modified aptamers exhibited stronger binding activity compared to unmodified aptamers or those modified with fluorine or methoxy alone. In Figures 14C-14D, 2'-fluoro-O-methyl (2'-F-O)-modified aptamers demonstrated enhanced binding activity relative to unmodified aptamers, while negative controls exhibited no binding. For the RBD recombinant proteins of both variants (Figures 14E-14F), modified aptamers displayed dose-dependent binding activity. When non-biotinylated aptamers ("cold-apt") were added, dose-dependent competitive binding activity was observed.

Based on the aforementioned results, further live virus neutralization experiments were conducted on the fluorinated and methoxy-co-modified aptamers against SARS-CoV-2 Omicron BA.1 and BA.2. As shown in Figures 15-17, all modified aptamers demonstrated favorable viral neutralization activity.

Following these findings, additional live virus neutralization experiments were performed using various PEG- and cholesterol-modified aptamers against SARS-CoV-2 Omicron BA.2. The results presented in Figure 18 indicated that all modified aptamers exhibited improved viral neutralization activity. Specifically, aptamer #5-2-15 displayed enhanced potential in live virus neutralization, prompting further chemical modifications of this candidate. As illustrated in Figures 19-21, the modified aptamers showed superior viral neutralization activities.

To verify the binding specificity of the aptamers, parallel live virus neutralization assays were performed using the individual chemical modifiers (cholesterol, PEG40K, and cholesterol-PEG40K) without aptamer conjugation. The results shown in Figures 22-24 revealed no detectable viral inhibition activity, indicating that the observed neutralization effects were not attributable to the isolated chemical modifiers (cholesterol, PEG40K, or cholesterol-PEG40K) themselves.

### Example 9. Prophylactic and Therapeutic Effects of Modified RNA Aptamers Against Viruses

To validate the *in vivo* neutralization activity of the screened and modified aptamers against live viruses, and to simulate their prophylactic and therapeutic effects, animal experiments were conducted based on previous findings. The chemically modified aptamers Chol-PEG40K-#5-2-15-2'-F-O and Chol-PEG6-#5-2-15-2'-F-O were tested for their prophylactic and therapeutic efficacy against SARS-CoV-2 Omicron BA.2 live virus in animals. As shown in Figure 25, the results demonstrated that the Chol-PEG40K-#5-2-15-2'-F-O RNA aptamer exhibited significant prophylactic and therapeutic effects at both 24 and 48 hours post-infection *in vivo.*

As illustrated in Figure 26, the Chol-PEG6-#5-2-15-2'-F-O aptamer also displayed prophylactic and therapeutic activity against SARS-CoV-2 Omicron BA.2 infection at 24 and 48 hours post-infection. Notably, compared to its efficacy at 48 hours, the Chol-PEG6-#5-2-15-2'-F-O aptamer demonstrated superior prophylactic and therapeutic effects at 24 hours post-infection with the live virus.

### Example 10. Applied Exploration of the CRISmers System

To expand the application scope of the CRISmers system, experiments were conducted involving the substitution of different CRISPR/Cas systems, various selection markers, and diverse screening hosts.

As shown in Figure 27, after the dCas9 in the CRISmers system was replaced with dCasMINI-V4, the affinity binding capacity of the aptamer to the target protein was still effectively converted into luciferase expression capability, activating luciferase production. This demonstrated that the CRISmers system of the present invention could be adapted to different CRISPR/Cas systems.

As illustrated in Figure 28, when the puromycin reporter gene in the CRISmers system was replaced with a green fluorescent protein (GFP) reporter gene, the results revealed that the aptamer's affinity binding to the target protein remained capable of driving GFP expression, activating GFP production. These findings confirmed that the CRISmers system could utilize distinct selection markers.

As shown in Figure 29, after the original puromycin reporter gene in the CRISmers system was replaced with an *E. coli* kanamycin reporter gene and transformed into *E. coli,* the affinity binding capability of the aptamer to the target protein was converted into the expression of the kanamycin reporter gene, reflected by bacterial viability under kanamycin selection. The results demonstrated that in the CRISmers system transformed into *E. coli,* the interaction between aptamer #5-2-15 and the SARS-CoV-2 RBD target protein was translated into kanamycin resistance, enabling *E. coli* growth on LB agar plates containing 100 µg/ml kanamycin. Similarly, the interaction between aptamer #2-1-18 and the SARS-CoV-2 RBD target protein conferred kanamycin resistance, supporting *E. coli* growth on LB agar plates containing 75 µg/ml kanamycin. This confirmed that the CRISmers system of the present invention could be applied to diverse host screening cells.

As illustrated in Figure 31, the screening components of the CRISmers system were integrated into a single vector. The target protein of interest was inserted into the vector for screening using the second restriction endonuclease EcoRI, while the random-sequence library was inserted into the vector via the first restriction endonuclease BsmBI, following the methodology for CRISPR sgRNA library construction, to generate the final screening library. The final library was introduced into *E. coli* screening hosts via electroporation. After ampicillin-based enrichment and kanamycin-based selection, *E. coli* colonies resistant to both ampicillin and kanamycin were collected. Plasmids were extracted from these colonies for downstream sequencing and validation processes.

It should be understood that, although the present invention has been specifically disclosed through preferred embodiments and optional features, modifications, refinements, and variations to the invention disclosed herein may be made by those skilled in the art, and such modifications, refinements, and variations are deemed to fall within the scope of the present invention. The materials, methods, and examples provided herein are representative of and illustrative for the preferred embodiments and are not intended to limit the scope of the invention.

## Claims

1. A system for screening an RNA aptamer against a target protein, comprising:
(i) a guide RNA comprising a recognition sequence and a nucleic acid aptamer random library having a predetermined length;
(ii) a target sequence complementary to the recognition sequence of the guide RNA;
(iii) a selection marker located downstream of the target sequence and comprising a basal promoter and a selection marker gene;
(iv) a fusion protein comprising the target protein and a transcriptional activation module, wherein a binding of the target protein to an RNA aptamer of the nucleic acid aptamer random library results in recruitment of the transcriptional activation module to the selection marker;
(v) a dCas protein specifically recognizing the target sequence under the guidance of the guide RNA; and
(vi) a screening cell.

2. The system of claim 1, wherein the nucleic acid aptamer random library consists of single-stranded oligonucleotide random sequences having 8 to 60 bases in the order of 10⁵ to 10³⁶.

3. The system of claim 1, wherein the nucleic acid aptamer random library is inserted into a loop on the guide RNA scaffold.

4. The system of claim 1, wherein the target sequence is a heterologous sequence and/or an artificial sequence.

5. The system of claim 4, wherein the target sequence comprises a plurality of copies of a gLuc sequence or Tet sequence.

6. The system of claim 1, wherein the selection marker gene is a luciferase gene, a fluorescent protein gene or an antibiotic resistance gene.

7. The system of claim 1, wherein the transcriptional activation module comprises a plurality of transcription activation factors.

8. The system of claim 7, wherein the transcriptional activation module comprises VP64, P65 and HSF1.

9. The system of claim 1, wherein the basal promoter is selected from mini-promoter-1 (SEQ ID NO: 28), mini-promoter-2 (SEQ ID NO: 27), mini-TK promoter (SEQ ID NO: 29), mini-CMV promoter (SEQ ID NO: 30) and Crystallin basal promoter (SEQ ID NO: 31).

10. The system of claim 1, wherein the dCas protein is a dCas9 protein or dUn1Cas12f1 protein.

11. The system of claim 1, comprising a viral expression vector, wherein the viral expression vector comprises the guide RNA and a nucleic acid encoding the fusion protein; and wherein the screening cell expresses the dCas protein and comprises the target sequence and the selection marker.

12. The system of claim 11, wherein the viral expression vector is a lentiviral expression vector.

13. The system of claim 12, wherein the lentiviral expression vector further comprises a first promoter operably linked to the guide RNA and a second promoter operably linked to the nucleic acid encoding the fusion protein.

14. The system of claim 13, wherein the nucleic acid aptamer random library is inserted into a scaffold of the guide RNA through a first restriction enzyme.

15. The system of claim 13, wherein a nucleic acid encoding the target protein is operably linked to the second promoter through a second restriction enzyme.

16. The system of claim 15, wherein the lentiviral expression vector comprises a polyT downstream of the guide RNA.

17. The system of claim 16, wherein the lentiviral expression vector comprises a nuclear localization sequence (NLS).

18. The system of claim 17, wherein the NLS comprises a first NLS downstream of the second promoter and a second NLS downstream of the nucleic acid encoding the fusion protein.

19. The system of claim 18, wherein the lentiviral expression vector comprises P2A, an antibiotic resistance gene and a post-transcription regulation element downstream of the second NLS.

20. The system of claim 19, wherein the P2A is linked to the second NLS in the upstream and the antibiotic resistance gene in the downstream, respectively, through a linker; and the nucleic acid encoding the target protein is linked to the first NLS in the upstream and a nucleic acid encoding the transcription activation factor in the downstream, respectively, through a linker.

21. The system of claim 1, wherein the screening cell is a eukaryotic or prokaryotic cell.

22. The system of claim 21, wherein the screening cell is a prokaryotic cell and the system comprises one or more plasmid vectors comprising one or more selected from a group consisting of: the guide RNA; the target sequence and the selection marker; a nucleic acid sequence encoding the fusion protein; and a nucleic acid sequence encoding the dCas protein.

23. The system of claim 22, wherein the system comprises a single plasmid vector comprising the guide RNA, the target sequence, the selection marker, and nucleic acid sequences encoding the fusion protein and the dCas protein.

24. The system of claim 1, wherein the target protein is derived from a virus, a bacterial, a fungus or an animal.

25. The system of claim 1, wherein the target protein is derived from a human.

26. The system of claim 1, wherein the target protein is a green fluorescent protein or is derived from a RBD region of S1 protein from SARS-CoV-2.

27. A method for screening an RNA aptamer against a target protein, comprising:
(1) providing a screening cell, wherein the screening cell comprises a guide RNA, a target sequence, a selection marker, a fusion protein and a dCas protein, wherein the guide RNA comprises a recognition sequence and a nucleic acid aptamer random library having a predetermined length; the target sequence is complementary to the recognition sequence of the guide RNA; the selection marker is downstream of the target sequence and comprises a basal promoter and a selection marker gene; the fusion protein comprises the target protein and a transcriptional activation module, wherein a binding of the target protein to an RNA aptamer of the nucleic acid aptamer random library results in recruitment of the transcriptional activation module to the selection marker; and the dCas protein specifically recognizes the target sequence under the guidance of the guide RNA;
(2) screening the screening cell using the selection marker gene;
(3) collecting the screening cell that expresses the selection marker gene;
(4) lysing the screening cell, specifically amplifying a region containing the nucleic acid aptamer, and obtaining sequence information regarding the nucleic acid aptamer by sequencing.

28. The method of claim 27, wherein step (4) further comprises an analysis and verification of the nucleic acid aptamer as obtained.

29. The method of claim 27, wherein the screening cell is obtained by transforming one or more plasmid vectors into a prokaryotic cell, wherein the one or more plasmid vectors comprise one or more selected from a group consisting of: the guide RNA; the target sequence and the selection marker; a nucleic acid sequence encoding the fusion protein; and a nucleic acid sequence encoding the dCas protein.

30. The method of claim 29, wherein the screening cell is obtained by transforming a single plasmid vector into a prokaryotic cell, wherein the single plasmid vector comprises the guide RNA, the target sequence, the selection marker, and nucleic acid sequences encoding the fusion protein and the dCas protein.

31. The method of claim 29, wherein the prokaryotic cell is *E. coli.*

32. The method of claim 27, the screening cell is obtained through the following steps:
(1.1) providing a cell that expresses the dCas protein, wherein the cell further comprises the target sequence and the selection marker;
(1.2) providing a viral expression vector comprising a nucleic acid encoding the guide RNA and the fusion protein; and
(1.3) packaging the viral expression vector and infecting the cell with the packaged viral expression vector.

33. The method of claim 32, further comprising
(5) subcloning the sequence of the nucleic acid aptamer obtained in step (4) to the viral expression vector obtained in step (1.2) and repeating steps (1.3) and steps (2) to (4).

34. The method of claim 33, wherein step (5) is repeated 1 to 3 times.

35. The method of claims 33 or 34, wherein virus infection is performed at an MOI of 3 to 5 when step (1.3) is initially performed, and at an MOI of 0.1 to 0.3 when step (1.3) is re-performed.

36. The method of claims 33 or 34, wherein in step (1.3) a virus titer of the packaged viral expression vector is determined using an antibiotic.

37. The method of claim 32, wherein the viral expression vector is lentiviral expression vector.

38. The method of claim 32, wherein the cell is a HEK293T cell.

39. The method of claim 27, wherein the dCas protein is a dCas9 protein or dUn1Cas12f1 protein.

40. The method of claim 27, wherein the basal promoter is selected from mini-promoter-1 (SEQ ID NO: 28), mini-promoter-2 (SEQ ID NO: 27), mini-TK promoter (SEQ ID NO: 29), mini-CMV promoter (SEQ ID NO: 30) and Crystallin basal promoter (SEQ ID NO: 31).

41. The method of claim 27, wherein the target protein is derived from a virus, a bacterial, a fungus or an animal.

42. The method of claim 27, wherein the target protein is derived from a human.

43. The method of claim 27, wherein the target protein is derived from a RBD region of S1 protein from SARS-CoV-2.

44. An RNA aptamer specifically binding to S1 protein of a SARS-CoV-2 virus, wherein the RNA aptamer comprises a random region in the middle flanked by constant sequences at both ends, wherein the constant sequences are complementary to each other, and the random region comprises a nucleic acid sequence set forth in any one of SEQ ID NOs: 1 to 7.

45. The RNA aptamer of claim 44, wherein the random region comprises a nucleic acid sequence set forth in SEQ ID NO: 2 or 7.

46. The RNA aptamer of claim 44, wherein the constant sequence at 5' end of the RNA aptamer comprises a nucleic acid sequence set forth in SEQ ID NO: 8.

47. The RNA aptamer of claim 44, wherein the constant sequence at 3' end of the RNA aptamer comprises a nucleic acid sequence set forth in SEQ ID NO: 9.

48. The RNA aptamer of claim 44, wherein the RNA aptamer is subject to one or more modifications selected from a group consisting of fluorine substitution, O-methylation, PEGylation and cholesterol-PEGylation.

49. The RNA aptamer of claim 48, wherein the PEGylation is selected from hexapolyethylene glycol to a PEG having a 40 kD molecule weight.

50. The RNA aptamer of claim 44, wherein the SARS-CoV-2 virus is a SARS-CoV-2 wild-type virus, a delta variant, an Omicron BA.1 variant, or an Omicron BA.2 variant.

51. A detection reagent or kit for detecting a SARS-CoV-2 virus, wherein the detection reagent or kit comprises an RNA aptamer of any one of claims 44 to 50.

52. A pharmaceutical agent for neutralizing a SARS-CoV-2 virus in a subject, wherein the pharmaceutical agent comprises an RNA aptamer of any one of claims 44 to 50.

53. The pharmaceutical agent of claim 52, further comprising a viral or non-viral vector, wherein the vector comprises or carries the RNA aptamer specifically binding to S1 protein from the SARS-CoV-2 virus.

54. A pharmaceutical composition comprising the pharmaceutical agent of claim 52 or 53 and a pharmaceutically acceptable excipient.

55. Use of an RNA aptamer of any one of claims 44 to 50 in the preparation of a medicament for prevention or treatment of SARS-CoV-2 virus infection.
